(19) Europäisches Patentamt
European Patent Office
Office européen des brevets

(11) **EP 4 101 471 A1**

(12) **EUROPEAN PATENT APPLICATION**
published in accordance with Art. 153(4) EPC

(43) Date of publication:
**14.12.2022  Bulletin 2022/50**

(21) Application number: **20901619.5**

(22) Date of filing: **09.12.2020**

(51) International Patent Classification (IPC):
**A61K 47/59** (2017.01)    **A61K 31/704** (2006.01)
**A61K 31/282** (2006.01)    **A61K 45/08** (2006.01)
**A61K 41/00** (2020.01)    **A61K 9/51** (2006.01)
**A61P 35/00** (2006.01)    **B82Y 5/00** (2011.01)

(52) Cooperative Patent Classification (CPC):
**A61K 9/51; A61K 31/00; A61K 31/282;
A61K 31/704; A61K 41/00; A61K 47/59;
A61P 35/00; B82Y 5/00**

(86) International application number:
**PCT/IB2020/061716**

(87) International publication number:
**WO 2021/124028 (24.06.2021 Gazette 2021/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.12.2019  MX 2019015508**

(71) Applicant: **Universidad de Guadalajara
44100 Guadalajara, Jalisco (MX)**

(72) Inventors:
• **DANERI NAVARRO, Adrián**
  **Guadalajara, Jalisco,  44340 (MX)**
• **TOPETE CAMACHO, Antonio**
  **Zapopan, Jalisco, 45190 (MX)**
• **ROSAS ESCAREÑO, Abraham Noé**
  **Guadalajara, Jalisco, 44940 (MX)**

(74) Representative: **Hoffmann Eitle
Hoffmann Eitle S.L.U.
Paseo de la Castellana 140, 3a planta
Edificio LIMA
28046 Madrid (ES)**

(54)    **NANOPARTICLES FOR CANCER TREATMENT**

(57)    The present invention is related to stimulated-release photoresponsive nanoparticles and their use as a new strategy to provide an antitumor treatment for breast cancer with increased specificity. Natural Killer (NK) cells with biocompatible stimulated-release photoresponsive nanoparticle-loaded cytotoxic granules are described, as well as methods for their preparation.

Fig. 1

**Description**

**FIELD OF THE INVENTION**

The present invention is related to nanoparticles for cancer treatment, more specifically with stimulated-release photoresponsive nanoparticles and their use for cancer treatment.

**BACKGROUND OF THE INVENTION**

Cancer is one of the leading causes of death worldwide and represents a serious public health problem. Despite advances in cancer treatment, through new strategies based on precision medicine, tumors in advanced stages, resistance to treatment, and undesirable effects continue to be a great challenge for humanity.

It is for the above that new specific therapies with greater effectiveness, less resistance potential, and side effects associated with cancer treatment are currently being implemented. One of the main problems with cancer treatments is that they affect not only cancer cells but healthy ones as well, and for this reason, the search continues to find therapies that only affect cancer cells.

Naturally, cytotoxic lymphocytes and specifically natural killer (NK) cells act against tumor cells. NK cells recognize a cancer cell, adhere to it, form an immunological synapse with the cancer cell and inject cytotoxic granules, which are substances designed to kill cells. As NK cells can recognize and attack tumor cells, they are very useful for localized cancer treatment. However, when the cancer is advanced or the patient's immune system is not competent enough, NK cells cannot destroy all tumor cells, and cancer progresses.

Various methods have been implemented to modify the cells of the immune system by strengthening them to help in the destruction of cancer cells. One of these methods is indicated in the document WO2016145578, which describes a method for treating cancer; in which T cells are extracted from the patient and loaded with antitumor peptides.

Then, the T cells are activated to act against cancer cells, and are reinjected into the patient. Another document that describes a similar process is EP2398466, where antineoplastic agents are deposited in NK cells extracted from a patient so that they are released into tumor cells once they are injected again. In both cases, the aim is for T or NK cells to transport antineoplastic agents to tumor cells in order to achieve localized treatment and avoid damage to healthy cells.

On the other hand, nanomedicine is responsible for designing, manufacturing and implementing intelligent nanometric-sized systems that combine functions such as the diagnosis of diseases or the transport and controlled release of drugs. According to this, it is known that nanomedicine is a promising therapeutic alternative for cancer treatment, more specifically due to its potential for providing the patient with efficient targeted systems that allow the treatment to preferentially affect cancer cells, reducing the adverse effects on healthy cells and tissues.

Recently, various cancer treatments that implement nanomedicine have been developed. In some cases, nanoparticles that have similar functions to those of NK cells have been designed, they identify cancer cells and deposite some therapeutic substance. For example, the document EP1917004. However, when injecting said nanoparticles into a patient, it cannot guarantee that the therapeutic substance will act directly with the tumor, and the therapeutic substances could spread to other parts of the body, causing harm to the patient.

Also, nanoparticles that can activate or enhance the reaction of T cells with a tumor have been developed. These nanoparticles carry an antigen that provokes an immune response by T cells, forcing them to act against the tissue where the nanoparticles are deposited. Some examples of documents describing antigen-carrying nanoparticles provoking an immune response by T cells are EP2736537, EP2637700 and EP2970907. In these cases, it may happen that by complicating the localized deposition of nanoparticles in the tumor area, they attract T cells to healthy patient tissues, causing a serious health problem.

Another form of nanotherapy that has been implemented is the one which injects the patient with nanoparticles loaded with antineoplastic agents, as indicated in documents EP1917004, EP2508207 and WO2012142669. However, as indicated above, it is difficult achieving that nanoparticles are deposited specifically in the area of the body where the cancerous tumor is, and it is not detected that the release of the drug contained in the nanoparticles is controlled when they are in the tumor, which could cause the drug to either be released in an area that does not have cancerous tissue or simply never release the drug when it reaches the tissue where it should be deposited.

One way to ensure that nanoparticles loaded with antineoplastic agents are transported directly to tumor cells is by binding them to the cell membrane of an NK cell, as described in document EP2398466. For increasing the chances that the NK cell detects tumor cells, it is activated before it is delivered to the patient. In this document, there is no control of the release of the antineoplastic agents, and as mentioned before, the agents could detach from the cell membrane of the NK cell and act in other parts of the human body, causing harm to the patient.

To achieve a targeted treatment through nanotherapy, various methods have been developed to treat cancer in which nanoparticles are administered to a patient for increasing the immunogenicity of tumors through an external stimulus. An example is the document EP2680919, which describes a method consisting of the preparation of carbon nanotubes loaded with immunoadjuvants. Once the nanotubes are prepared, they are administered to the tumor and the tumor is subsequently irradiated with a laser to produce a photothermal effect that releases the immunoadjuvants and causes a photothermal effect that destroys cancer cells. Another similar treatment is described in the document EP1834646, where the patient is administered with semiconducting metal particles, which subsequently receive ultrasonic radiation and destroy cancer cells. Another method is described in the document EP1841468, where the patient is given nanoparticles that agglomerate around the cancer cells, forming a cage. These nanoparticles are irradiated with lasers and damage cancerous tissue. All these methods require an external stimulus to activate the effect of the nanoparticles, helping the effect to be localized and not damage other tissues. However, in all previous cases, the nanoparticles must be deposited directly into the tumor or allow the nanoparticles flowing through the bloodstream with the expectation that they will be deposited at the desired site. This can be a problem for cases in which the tumors are in places that are difficult to access, since the particles cannot be deposited directly on the tumor. Also, if the nanoparticles are injected into the bloodstream, there is no guarantee that they will reach the tumor or act properly.

Although many cancer treatments have been identified, which take advantage of the tumor-targeting, and the attacking to the T cells, specifically with NK cells, and treatments that use nanoparticles to target a tumor in various ways. It was not detected any treatment describing nanoparticles loaded with antineoplastic agents that can be transported inside NK cells, and that can be locally activated to release antineoplastic agents and simultaneously cause localized hyperthermia in a malignant tumor.

Therefore, the aim is to find a nanoparticle loaded with antineoplastic agents capable of coupling to the cytotoxic granules of an NK cell and activated by laser radiation to release antineoplastic agents and cause localized hyperthermia in a cancerous tumor.

## OBJETS OF THE INVENTION

Considering the nonspecificity associated with systemic chemotherapeutic treatments for breast cancer, it is an object of the present invention to establish a new strategy to provide an antitumor treatment for breast cancer with increased specificity.

Another object of the present invention is to make use of photoresponsive biocompatible nanoparticles with multimodal therapeutic activity that can be activated spatio-temporally through infrared radiation, functionalized with monoclonal antibodies to localize in the cytotoxic granules of NK cells.

Another object of the present invention is to load NK cells with multimodal nanoparticles under standard culture conditions without affecting their recognition capacities or their effector cytotoxic mechanisms against tumor cells.

Another object of the present invention is to take advantage of the natural tropism of NK cells towards tumor cells and their degranulation mechanisms to deliver nanoparticles to tumor cells.

Finally, it is an object of the present invention to combine the biological specificity of NK cells to deliver nanoparticles to tumor cells and the spatio-temporal activation of the therapeutic activity of nanoparticles through infrared radiation after ensuring the interaction of NK cells with tumor cells, and the release of nanoparticles in tumor cells, allowing to increase the magnitude levels in specificity in the treatment of breast cancer.

## BRIEF DESCRIPTION OF THE INVENTION

The present invention solves all the problems and disadvantages identified in the background of this document.

A first aspect of the present invention refers to a stimulated-release photoresponsive nanoparticle which comprises a drug encapsulated in a biodegradable synthetic polymer coated with a photosensitizing agent.

A second aspect of the invention refers to a method for obtaining photoresponsive nanoparticles with stimulated release, comprising a stage for forming biodegradable polymeric nanoparticles with encapsulated drugs and, a stage for coating those nanoparticles with a photosensitizing agent.

One more aspect of the present invention relates to a biocompatible stimulated-release photothermal nanoparticle comprising a stimulated-release photoresponsive nanoparticle coupled to at least one humanized monoclonal antibody of the IgG type.

Yet another aspect of the present invention relates to a method for obtaining biocompatible stimulated-release photoresponsive nanoparticles that comprises binding humanized IgG-type monoclonal antibodies to stimulated-release photoresponsive nanoparticles through a covalent bond.

An additional aspect of the present invention refers to Natural Killer (NK) cells with cytotoxic granules loaded with biocompatible stimulated-release photoresponsive nanoparticles.

Another additional aspect of the present invention relates to a method for introducing the biocompatible stimulated-release photoresponsive nanoparticles to the cytotoxic granules of an NK cell which comprises incubating NK cells under standard culture conditions in the presence of biocompatible stimulated-release photoresponsive nanoparticles.

One more aspect of the present invention relates to an injectable suspension comprising NK cells with cytotoxic granules loaded with biocompatible stimulated-release photoresponsive nanoparticle and a suspension medium.

Another aspect of the present invention relates to a method for obtaining an injectable suspension comprising NK cells with cytotoxic granules loaded with biocompatible stimulated-release photoresponsive nanoparticles, comprising a stage of molecular stimulation of NK cells with cytotoxic granules loaded with biocompatible stimulated-release photoresponsive nanoparticles to obtain activated NK cells; and a stage of mixing the activated NK cells with a suspension medium.

One more aspect of the present invention comprises a method for depositing biocompatible stimulated-release photoresponsive nanoparticles in tumor tissue comprising a step of obtaining an injectable suspension with activated NK cells; a stage of contact of the activated NK cells with the tumor tissue, where the activated NK cells are administered by the injectable suspension; and a release stage of cytotoxic granules loaded with the biocompatible stimulated-release photoresponsive nanoparticles contained in the activated NK cells.

One more aspect of the present invention refers to a method for releasing a drug of a biocompatible stimulated-release photoresponsive nanoparticle that comprises exposing the stimulated-release photoresponsive nanoparticle to a source of electromagnetic radiation until obtaining an increase in the temperature from 23 to 25°C.

Another aspect of the present invention relates to the use of biocompatible stimulated-release photoresponsive nanoparticles to treat cancerous tumors by the deposition of biocompatible stimulated-release photoresponsive nanoparticles in tumor tissue, followed by exposing them to a source of electromagnetic radiation to cause the release of the drug contained in the nanoparticles inside the tumor tissue.

## DESCRIPTION OF THE FIGURES

The novel aspects that are considered characteristic of the present invention will be established with particularity in the appended claims. However, its characteristics and advantages will be better understood in the examples when they are read with the attached figures, where:

Figure 1 shows the UV-Vis absorption spectra of AuNPs, PEG-AuNPs, and anti-GZMB AuNPs obtained in Example 1 according to an embodiment of the present invention.

Figure 2 shows the micrographs of the AuNPs obtained in Example 1 according to one embodiment of the present invention by transmission electron microscopy.

Figure 3 shows the heating and cooling profile obtained from anti-GZMB AuNPs obtained in Example 1 after serial irradiation for 180 min with infrared light at 800 nm in an aqueous medium in accordance with one embodiment of the present invention.

Figure 4 shows the UV-Vis absorption spectra of PLGA/DOX/Ch, PLGA/DOX/Ch/ICG and PLGA/DOX/Ch/ICG NPs obtained in Example 4 in accordance with one embodiment of the present invention.

Figure 5 shows atomic force microscopy (left) and scanning electron microscopy (right) micrographs and a histogram of the size distribution of the PLGA NPs obtained in Example 4 in accordance with one embodiment of the present invention.

Figure 6 shows the heating and cooling profile of anti-GZMB PLGA/DOX/Ch/ICG NPs obtained in Example 4 after irradiation with infrared light at 800 nm for 150 min in an aqueous medium according to one embodiment hereof invention.

Figure 7 shows the release profile of DOX contained in anti-GZMB PLGA/DOX/ICG/NPs in the absence (-) and presence (+) of infrared light irradiation in accordance with one embodiment of the present invention.

Figure 8 shows the effect on cell viability of 800 nm infrared light irradiation on triple-negative (HCC70) and HER2+ (HCC1954) breast cancer cells in accordance with one embodiment of the present invention.

Figure 9 shows the dose-response curves to estimate the effective dose 50 (EDso) of anti-GZMB AuNPs and anti-GZMB PLGA/DOX/Ch/ICG NPs obtained in Examples 1 and 4, on triple-negative (HCC70) and HER2+ (HCC1954) breast cancer cells in the presence (+) and absence (-) of irradiation with infrared light at 800 nm according to two embodiments of the present invention.

Figure 10 shows the increase in the production of superoxide anion ($O_2^{\bullet-}$) and singlet oxygen ($^1O_2$) derived from treatment with anti-GZMB AuNPs and anti-GZMB PLGA/DOX/Ch/ICG NPs in the absence (-) and presence (+) irradiation with infrared light on triple-negative (HCC70) and HER2+ (HCC1954) breast cancer cells.

Figure 11 shows the viability effects of anti-GZMB AuNPs and anti-GZMB PLGA/DOX/Ch/ICG NPs obtained in Examples 1 and 4 on NK cells of the NKL cell line according to two embodiments of the present invention.

Figure 12 shows the internalization of anti-GZMB AuNPs and anti-GZMB PLGA/DOX/Ch/ICG NPs obtained in Examples 1 and 4 in NKL cells under standard culture conditions after exposure for 24 hours according to two modalities of the present invention.

Figure 13 shows the internalization of AuNPs and anti-GZMB PLGA/DOX/Ch/ICG NPs obtained in Examples 1 and 4 in NKL cells under standard culture conditions after exposure for 24 hours according to two embodiments of the present invention.

Figure 14 shows the exocytosis of anti-GZMB PLGA/DOX/Ch/ICG NPs after exposure to nanoparticles for 24 hours in accordance with one embodiment of the present invention.

Figure 15 shows the results of the quantification of anti-GZMB AuNPs and anti-GZMB PLGA/DOX/Ch/ICG NPs obtained from subcellular fractionation and enrichment of CD63+ lysosomes.

Figure 16 shows representative histograms and average mean fluorescence intensity of NKp30, NKp44, NKp46, and NKG2D cellular cytotoxicity receptors expression of NKL cells after exposure to anti-GZMB AuNPs and anti-GZMB PLGA/DOX/Ch/ICG NPs for 24 hours obtained by flow cytometry.

Figure 17 shows representative histograms and average mean fluorescence intensity of expression of TNF-$\alpha$ and IFNy production by NKL cells after exposure to anti-GZMB AuNPs and anti-GZMB PLGA/DOX/Ch/ICG NPs for 6 hours obtained by flow cytometry.

Figure 18 shows representative histograms and average mean fluorescence intensity of expression of TNF-$\alpha$ and IFNy production by NKL cells loaded with anti-GZMB AuNPs and anti-GZMB PLGA/DOX/Ch/ICG NPs and stimulation with PMA and ionomycin for 6 hours.

Figure 19 shows representative histograms and average mean fluorescence intensity of CD107a expression after exposure of NKL cells loaded with anti-GZMB AuNPs, anti-GZMB PLGA/DOX/Ch/ICG NPs, or without nanoparticles with HCC70, HCC1954 and HCC1954 + TZB* cells for 4 hours.

Figure 20 shows representative histograms and average mean fluorescence intensity of ICG from PLGA/DOX/Ch/ICG NPs present in NKL cells at baseline (t=0) and after co-culture with HCC70, HCC1954, and HCC1954 + TZB* cells for 4 hours.

Figure 21 shows the internalization curves of anti-GZMB AuNPs and anti-GZMB PLGA/DOX/Ch/ICG NPs at 6, 9, 12, 18, and 24 hours under standard culture conditions with the doses selected in Example 10, according to two embodiments of the invention.

Figure 22 shows the basal specific cytotoxicity curves of NKL cells against triple-negative (HCC70) and HER2+ (HCC1954) breast cancer cells with and without TZB at different proportions of effector cells and target cells according to two modalities of the present invention.

Figure 23 shows the specific cytotoxicity of NKL cells loaded with anti-GZMB AuNPs, anti-GZMB PLGA/DOX/Ch/ICG NPs or nanoparticles without antibody against triple-negative (HCC70) and HER2+ (HCC1954) breast cancer cells with and without TZB in the absence (-) and presence (+) of infrared light irradiation according to two embodiments of the present invention.

Figure 24 shows a flow cytometric analysis of the cytotoxic activity of NKL cells loaded with anti-GZMB PLGA/DOX/Ch/ICG NPs and their degranulation against HCC70 cells.

Figure 25 shows a flow cytometric analysis of the cytotoxic activity of NKL cells loaded with anti-GZMB PLGA/DOX/Ch/ICG NPs and their degranulation against HCC1954 cells.

Figure 26 shows a flow cytometric analysis of the cytotoxic activity of NKL cells loaded with anti-GZMB PLGA/DOX/Ch/ICG NPs and their degranulation against HCC1954 + TZB* cells.

Figure 27 shows a flow cytometric analysis of the cytotoxic activity of NKL cells loaded with PLGA/DOX/Ch/ICG NPs without anti-GZMB monoclonal antibodies and their degranulation against HCC1954 + TZB* cells.

## DETAILED DESCRIPTION OF THE INVENTION

As mentioned in the background, the aggressiveness of existing cancer treatments has led to the search for developing technologies that achieve localized treatment of cancer. Among these technologies, it has been observed that the use of nanoparticles to carry out this type of treatment has increased. However, it has been observed that it is very complicated for those nanoparticles to be deposited only in cancer cells without having any risk of affecting healthy cells of the body, thus attempting the patient's health. For the previous reason, photoresponsive nanoparticles loaded with antineoplastic agents have been developed, which can be transported by Natural Killer (NK) cells and can localized release antineoplastic agents and simultaneously provoking localized hyperthermia in a cancerous tumor when stimulated.

Therefore, the present invention relates primarily to a stimulated-release photoresponsive nanoparticle comprising a drug encapsulated in a biodegradable synthetic polymer coated with a photosensitizing agent.

For purposes of the present invention, the term "photoresponsive nanoparticle" refers to nanoparticles which respond to a light stimulus, that response can be manifested by radiative phenomena (fluorescence) or non-radiative (increase in temperature and generation of reactive oxygen species).

Preferably, the stimulated-release photoresponsive nanoparticles have an average size between 50 nm and 200 nm.

In a preferred embodiment of the present invention, the drug is selected from doxorubicin, cisplatin, carboplatin, or a combination of the above. Preferably, the encapsulated drug is between 1% and 25% by weight relative to the weight of the stimulated-release photoresponsive nanoparticles.

Preferably in the present invention, the biodegradable synthetic polymer of the stimulated-release photoresponsive nanoparticles is Poly(lactic-co-glycolic acid) (PLGA) copolymer.

In a preferred embodiment, the photosensitizing agent coating the biodegradable synthetic polymer is indocyanine green (ICG).

ICG is a fluorescent molecule with very low toxicity, which has the ability to absorb and emit in the near-infrared spectral range, in other words, it is capable of converting incident photons into thermal energy, so it is possible to activate the release of the drug by thermal energy generated by illumination with a laser of suitable wavelength.

In addition, the applied thermal energy allows the stimulated-release photoresponsive nanoparticles to be used as biodegradable and biocompatible photoactivatable systems for the controlled release of antineoplastic agents, as hyperthermia-inducing agents, and as photosensitizers for photodynamic therapy. ICG has the ability to absorb and emit in the near-infrared spectral range, with an excitation wavelength around 790nm and an emission wavelength around 810nm. This fluorescent dye has been approved by the Food and Drug Administration (FDA).

A second aspect of the invention relates to a method for obtaining photoresponsive nanoparticles with stimulated release, comprising a stage for forming biodegradable polymeric nanoparticles with encapsulated drugs and a stage for coating those nanoparticles with a photosensitizing agent.

In a preferred embodiment of the present invention, the stage of forming the biodegradable polymeric nanoparticles with encapsulated drugs of the method for obtaining stimulated-release photoresponsive nanoparticles comprises the following steps: 1) dissolving the biodegradable synthetic polymer with a drug in an organic solvent; 2) add the organic phase containing the polymer and the drug to an aqueous phase; 3) form an emulsion; 4) evaporate the solvent; 5) centrifuge the mixture; and 6) washing with deionized water to remove excess of stabilizer and the unencapsulated drug.

In a preferred embodiment of the present invention, the organic solvent used in the step of dissolving the biodegradable synthetic polymer and the drug is selected from among dichloromethane, chloroform, and acetone.

In an optional embodiment, the aqueous phase may contain a stabilizer, which is selected from polyvinyl alcohol or copolymers, or mixtures of copolymers containing polyethylene glycol and polypropylene glycol.

Preferably, the emulsion is formed by any method known to those skilled in the art for mixing organic phases with aqueous phases. Sonication is preferably used.

In a preferred embodiment of the present invention, the evaporation of the solvent is carried out by stirring at room temperature.

In a preferred embodiment of the present invention, the mixture centrifugation step is carried out at a speed between 8,000 g and 10,000 g. More preferably, the centrifugation is carried out at 9,000 g.

Preferably, the step of centrifugation of the mixture is carried out at a temperature between 7 and 12 °C. More preferably, it is carried out at a temperature of 10°C.

In a preferred embodiment of the present invention, the stage of coating the biodegradable polymeric nanoparticles with encapsulated drugs with a photosensitizing agent of the method to obtain stimulated-release photoresponsive nanoparticles comprises the following steps: 1) invert the surface charge of the nanoparticles to achieve electrostatic physisorption through the formation of a layer of a cationic polymer leaving exposed -NH$_2$ groups on the surface of biodegradable polymeric nanoparticles with encapsulated drugs; 2) mixing the biodegradable polymeric nanoparticles with drugs encapsulated with a photosensitizing agent; 3) stimulate the interaction of biodegradable polymeric nanoparticles with drugs encapsulated with the photosensitizing agent by a moderate agitation; and 4) remove excess of photosensitizing agent by dialysis.

Preferably, the biodegradable polymeric nanoparticles with encapsulated drugs and the photosensitizing agent are mixed in a ratio between 0.2% and 5%.

In a preferable embodiment of the present invention, moderate stirring is carried out at room temperature for 12 hours.

In a preferred embodiment of the present invention, the formation of the cationic polymer layer that exposes -NH$_2$ groups on the surface of the biodegradable polymeric nanoparticles with encapsulated drugs is carried out by the layer-by-layer method. Preferably, the cationic polymer leaving exposed -NH$_2$ groups is selected from chitosan, cationic cellulose, poly(allylamine) or other cationic polymers, synthetic or natural.

One more aspect of the present invention relates to a biocompatible stimulated-release photoresponsive nanoparticle comprising a stimulated-release photoresponsive nanoparticle coupled to at least one humanized monoclonal antibody of the IgG type.

It is necessary for the stimulated-release photoresponsive nanoparticles to be biocompatible in order to be subsequently introduced into the cytotoxic granules of NK cells, as will be explained later.

Preferably, the humanized IgG monoclonal antibodies are selected from humanized IgG monoclonal antibodies specific for LAMP1 or granzyme B, or a mixture thereof.

Yet another aspect of the present invention relates to a method for obtaining biocompatible stimulated-release photoresponsive nanoparticles that comprises binding humanized IgG monoclonal antibodies to stimulated-release photoresponsive nanoparticles by a covalent bond.

In a preferred embodiment of the present invention, the stimulated-release photoresponsive nanoparticles bind to the humanized IgG monoclonal antibodies through the -NH$_2$ functional groups, which were generated during their preparation.

On the other hand, the -COOH ends of the humanized IgG monoclonal antibodies are preactivated prior to their union with biocompatible stimulated-release photoresponsive nanoparticles. The preactivation of the -COOH ends of the antibody is carried out by carbodiimide chemistry, adding 1-ethyl-3-(3-dimethylaminopropyl) carbodiimide (EDC), and sulfo-N-hydroxysuccinimide (sulfo-NHS) to an antibody solution.

The binding reaction is caused by mixing the stimulated-release photoresponsive nanoparticles with the humanized IgG monoclonal antibodies in a ratio of nanoparticle: antibody of 1:1 to 1:20. Preferably, the mixing is carried out using the microfluidic technique.

An additional aspect of the present invention relates to NK cells with cytotoxic granules loaded with biocompatible stimulated-release photoresponsive nanoparticles.

In a preferred embodiment of the present invention, the NK cells are extracted from the peripheral blood of the patient. NK cells transport biocompatible stimulated-release photoresponsive nanoparticles in the cytotoxic granules to tumor cells due to natural mechanisms of NK cell cytotoxicity that include cytokines, chemokines, chemokine receptors, and activating receptor ligands. NK cells allow the release of biocompatible stimulated-release photoresponsive nanoparticles because they have cytotoxic granules, the mechanism they use to kill is always the release of cytotoxic granules, therefore if the cytotoxic granules have biocompatible stimulated-release photoresponsive nanoparticles, the treatment will have a double effect of eliminating cancer cells, since the cytotoxic granules released through the natural mechanisms of the NK cell will have stimulated-release photoresponsive nanoparticles.

Another additional aspect of the present invention relates to a method for introducing the biocompatible stimulated-release photoresponsive nanoparticles to the cytotoxic granules of an NK cell comprising incubating NK cells under standard culture conditions in the presence of biocompatible stimulated-release photoresponsive nanoparticles.

The incubation consists of putting a quantity of biocompatible stimulated-release photoresponsive nanoparticles suspended in a culture medium in contact with NK cells to favor their interaction and the internalization of the nanoparticles. In a preferred embodiment of the present invention, the standard culture conditions are 37°C, 5% of $CO_2$, and sterile conditions.

In a preferred embodiment, the incubation is carried out until ensuring that the NK cells have incorporated a therapeutically effective amount of the biocompatible stimulated-release photoresponsive nanoparticles. Each NK cell incorporates between 8,000 and 12,000 biocompatible stimulated-release photoresponsive nanoparticles. The internalization of stimulated-release biocompatible photoresponsive nanoparticles can be evidenced by flow cytometry before being administered to the patient. Preferably, the incubation of the biocompatible stimulated-release photoresponsive nanoparticles and the NK cells is carried out for 12 hours.

A further aspect of the present invention relates to an injectable suspension comprising NK cells with cytotoxic granules loaded with biocompatible stimulated-release photoresponsive nanoparticles and a suspension medium, such as an injectable saline solution.

The injectable suspension will be injected into the patient. Even when stimulated-release photoresponsive nanoparticles can be administered directly into the bloodstream to reach tumor tissue, there is a risk of drug leakage and consequent damage to healthy tissues and cells. For this reason, the present invention focuses on the ability of NK cells to locate tumor tissue or tumor cells and attack them by releasing their cytotoxic granules, therefore, NK cells with cytotoxic granules loaded with biocompatible stimulated-release photoresponsive nanoparticles will allow simultaneously release cytotoxic granules and biocompatible stimulated-release photoresponsive nanoparticles in tumor tissue, avoiding the collateral damage of conventional chemotherapy.

For this reason, the present invention focuses on the ability of NK cells to locate tumor tissue or tumor cells and attack them by releasing their cytotoxic granules, therefore, NK cells with cytotoxic granules loaded with biocompatible stimulated-release photoresponsive nanoparticles will allow simultaneously release cytotoxic granules and biocompatible stimulated-release photoresponsive nanoparticles in tumor tissue, avoiding the collateral damage of conventional chemotherapy.

NK cells move towards tumor tissue by receptors that recognize chemokines produced both by the tumor tissue and, by other infiltrating cells of the immune system. Once in the tumor tissue, cancer cells can be recognized by NK cells through the expression of molecules such as MICA, MICB, B7-H6, BAG6/BAT3, and the loss of HLA molecules. These molecules are recognized through receptors expressed by NK cells such as NKp30, NKp44, NKp46, NKG2D and KIR.

Another aspect of the present invention relates to a method for obtaining an injectable suspension comprising NK cells with cytotoxic granules loaded with biocompatible stimulated-release photothermal nanoparticles comprising a stage of molecular stimulation of NK cells with cytotoxic granules loaded with biocompatible stimulated-release photoresponsive nanoparticles, so that overexpression of their recognition and cytotoxicity receptors is induced, in order to obtain activated NK cells; and a stage of mixing the activated NK cells with a suspension medium.

In a preferred embodiment of the present invention, the stage of molecular stimulation of NK cells with cytotoxic granules loaded with biocompatible stimulated-release photothermal nanoparticles comprises adding interleukins to the culture medium. Preferably, the interleukins are selected from IL-2, IL-15, IL-18, or mixtures thereof. Interleukins are proteins that act as activation signals for the expression of recognition receptors NKp30, NKp44, NKp46 and NKG2D, and serve to mature the machinery by which NK cells destroy cancer cells, such as Granzyme B and Perforin. Molecular stimulation of NK cells with interleukins is carried out for 72 hours at 37 °C and 5% of $CO_2$ under standard cell culture conditions.

The injectable suspension is injected into the patient.

One more aspect of the present invention comprises a method for depositing biocompatible stimulated-release photoresponsive nanoparticles in tumor tissue comprising a stage of obtaining an injectable suspension comprising activated NK cells; a stage of contact of the activated NK cells with the tumor tissue, where the activated NK cells are administered by the injectable suspension; and a release stage of the cytotoxic granules loaded with biocompatible stimulated-release photoresponsive nanoparticle contained in the activated NK cells.

In a preferred embodiment of the present invention, the stage of obtaining an injectable suspension comprising activated NK cells is carried out as previously described in this descriptive document.

The release stage of the cytotoxic granules loaded with biocompatible stimulated-release photoresponsive nanoparticles contained in the activated NK cells is carried out by the activated NK cells. The activated NK cells will recognize the tumor tissue in the patient through their intrinsic recognition mechanisms to later release the cytotoxic granules that contain the biocompatible stimulated-release photoresponsive nanoparticles into the tumor tissue and tumor cells.

One more aspect of the present invention refers to a method for the release of a drug from a stimulated-release photoresponsive nanoparticle that comprises exposing the stimulated-release photoresponsive nanoparticle to a source of electromagnetic radiation until obtaining an increase in temperature of 23 to 25 °C, which ensures the glassy transition of the polymer and the subsequent release of the drug.

In a preferred embodiment of the present invention, the source of electromagnetic radiation is a laser. More preferably, the source of electromagnetic radiation is a laser having a wavelength close to 800nm. In this case, the electromagnetic radiation serves to activate localized hyperthermia in the tumor tissue and release the drug from the stimulated-release photoresponsive nanoparticles, inducing apoptosis or necrosis of the tumor cells in the patient in a controlled manner.

Yet another aspect of the present invention relates to the use of stimulated-release photoresponsive nanoparticles for treating cancerous tumors by deposition of biocompatible stimulated-release photoresponsive nanoparticles in tumor tissue, followed by exposing them to a source of electromagnetic radiation to induce the release of the drug contained in the nanoparticles into that tumor tissue.

In an alternative embodiment of the present invention, NK cells with cytotoxic granules loaded with biocompatible stimulated-release photoresponsive nanoparticles that also comprise biocompatible metallic nanoparticles are described. In this way it is possible to obtain a better result in the treatment of cancerous tumors.

Metallic nanoparticles are versatile metallic nanostructures with multiple emerging applications in the biomedical area. Preferably the metal used is gold. Optionally, the hollow metallic nanoparticles are synthesized using the galvanic replacement method, which consists of generating silver seeds that serve as a template for the growth of the metal on this seed; thus, the silver serves as a sacrificial mold, since the metal is reduced on its surface and at the same time oxidizes the silver, thus giving rise to metallic nanoparticles.

Preferably, the biocompatible metallic nanoparticles have a spheroid morphology with an average diameter between 50 and 200 nm. More preferably, the biocompatible metallic nanoparticles have a hollow interior, and the thickness of the metal nanoshell is between 5 and 30 nm.

One of the main characteristics of biocompatible metallic nanoparticles is their ability to transform light energy into heat after being irradiated with an electromagnetic radiation source. In this way, introducing biocompatible metallic nanoparticles into the cytotoxic granules of NK cells, together with stimulated-release photoresponsive nanoparticles, can help in the treatment of cancerous tumors by applying a source of electromagnetic radiation and generating localized heat. Thus, the biocompatible metallic nanoparticles exert their cytotoxic effect through the local increase in temperature after being irradiated.

Preferably, the biocompatible metallic nanoparticles have a resonance plasmon located at 800 $\pm$ 10 nm.

In order to successfully introduce biocompatible metallic nanoparticles into the cytotoxic granules of NK cells, as in the case of stimulated-release photoresponsive nanoparticles, it is necessary to subject them to a treatment to biofunctionalize them, hence their biocompatibility.

In this case, the treatment consists of a PEGylation process, in which metallic nanoparticles are mixed with a polyethylene glycol (PEG) solution containing a -SH functional group at one end and a $-NH_2$ group. The mixture is moderately stirred for a period of time from 48 to 96 hours, preferably 72 hours.

After PEGylation, as in the case of photoresponsive stimulated-release nanoparticles, the metallic nanoparticles are bound to the humanized IgG monoclonal antibodies by the $-NH_2$ functional groups, which in this case are added during PEGylation, to form biocompatible metallic nanoparticles.

In the same way, in order to carry out the union of the humanized IgG monoclonal antibodies with the metallic nanoparticles, it is necessary to preactivate the -COOH ends of those antibodies. Preactivation of the -COOH ends of the antibody is carried out by carbodiimide chemistry, adding EDC and sulfo-NHS to an antibody solution.

Thus, the formation of the biocompatible metallic nanoparticles is carried out by mixing the previously PEGylated metallic nanoparticles with the IgG humanized monoclonal antibodies in a ratio of 1 to 20 antibodies per biocompatible metallic. Preferably, the mixture is carried out using the microfluidic technique to efficiently anchor these antibodies covalently to the previously PEGylated metallic nanoparticles through an amide bond.

In a preferred embodiment of the present invention, the appropriate pH to favor the formation of biocompatible metallic nanoparticles is between 4 to 6.

Once the biocompatible metallic nanoparticles are obtained, they are introduced into the cytotoxic granules of the NK cells following the same method described above for the biocompatible stimulated-release photoresponsive nanoparticles. Its use for the treatment of cancerous tumors is similar to that described for photoresponsive nanoparticles with stimulated-release by exposing them to the source of electromagnetic radiation.

The present invention will be better understood from the following examples, which are presented solely for illustrative purposes to allow a full understanding of the preferred embodiments of the present invention, without implying that there are no other modes not illustrated that can be carried out based on the detailed description above.

## EXAMPLE 1

The method for obtaining biocompatible gold nanoparticles (AuNPs) with spatio-temporally activatable photothermal activity through infrared light and their functionalization with anti-GZMB monoclonal antibodies in accordance with an embodiment of the present invention is described.

The synthesis of AuNPs was carried out through galvanic replacement reactions using silver nanoparticles (AgNPs) as a mold for the growth of the gold shell. AgNPs were synthesized using silver nitrate ($AgNO_3$) as ionic silver source, trisodium citrate ($Na_3C_6H_5O_7$) as stabilizing agent, and L-ascorbic acid ($C_6H_8O_6$) as reducing agent. The preparation of a batch of AuNPs begins with 30 mL of a 2.5 mM aqueous solution of trisodium citrate (Sigma-Aldrich® - S4641) and 1.0 mM of silver nitrate (Sigma-Aldrich® - 209139), to which added the necessary mass of ascorbic acid (Sigma-Aldrich® - A7506) dissolved in deionized water to bring the solution to a final concentration of 0.1 mM; after the addition of the reducing agent, the solution was allowed to react for 30 minutes at room temperature with constant magnetic stirring until a yellow-orange solution corresponding to the generation of an AgNPs colloid was obtained. After the generation of AgNPs, the galvanic replacement reaction was carried out. For this reaction, 30 mL of silver seeds were adjusted to a final concentration of 5.0 mM of hydroxylamine hydrochloride ($NH_2OH$-Cl) (Sigma-Aldrich® - 255580) and the suspension was left to react at room temperature with constant magnetic stirring for 5 minutes, after this time, the necessary volume of a 10.0 mM solution of $HAuCl_4$ (Sigma-Aldrich® - 254169) was added dropwise until a final concentration of 0.33 mM was obtained; after the addition of the gold solution, the suspension turned greenish blue, evidencing the formation of AuNPs. The solution was left to react for 10 min and after this time, the reducing activity of hydroxylamine was stopped by adding nitric acid ($HNO_3$) (J.T. Baker - UN2031) adjusting the suspension of AuNPs to a final concentration of 3.0 mM (pH 5.5). Immediately, the surface of the gold nanoparticles was modified. To modify the surface of the nanoparticles, and increase their biocompatibility, were added 10 mg of SH-PEG-NH2 PEG /P.M. 3400 Da (Laysan Bio, Inc) dissolved in deionized water to 30 mL of newly synthesized AuNPs and the suspension was kept under constant magnetic stirring for 72 h at room temperature protected from light. After 72 h, a gradual low-speed centrifugation (350 g, 90 min) was performed in order to separate the AgCl crystals generated *in situ* during the replacement reaction; after centrifugation, the supernatant was carefully recovered and the pellet containing the AgCl crystals was discarded. The recovered supernatant (containing PEG-AuNPs without AgCl) was washed twice by centrifugation at 3,000 g to eliminate the excess of non-bound PEG, subsequently, the PEG-AuNPs were resuspended in PBS and the surface was functionalized anti-GZMB monoclonal antibodies, with the number of antibodies per AuNPs being able to range from 1 to 20.

Functionalization was carried out through microfluidics and covalent crosslinking between the $-NH_2$ terminal ends of PEG and the -COOH end of the Fc fraction of the monoclonal antibody. Prior to cross-linking, the monoclonal antibody was pre-activated with EDC and sulfo-NHS in MES buffer (Sigma-Aldrich - M8250) at a pH 6.0 to form amino-reactive ends in the -COOH region of the antibody to subsequently covalently bind them to the terminal $-NH_2$ ends present on the surface of the nanoparticle; This was achieved through the use of a microchip that allowed the controlled interaction of the monoclonal antibody with the nanoparticles in a laminar interface. Functionalization with anti-GZMB monoclonal antibodies in each nanoparticle was confirmed using the NanoOrange® protein quantification kit (Invitrogen™ - N666), with a range of 1 to 20 antibodies per nanoparticle.

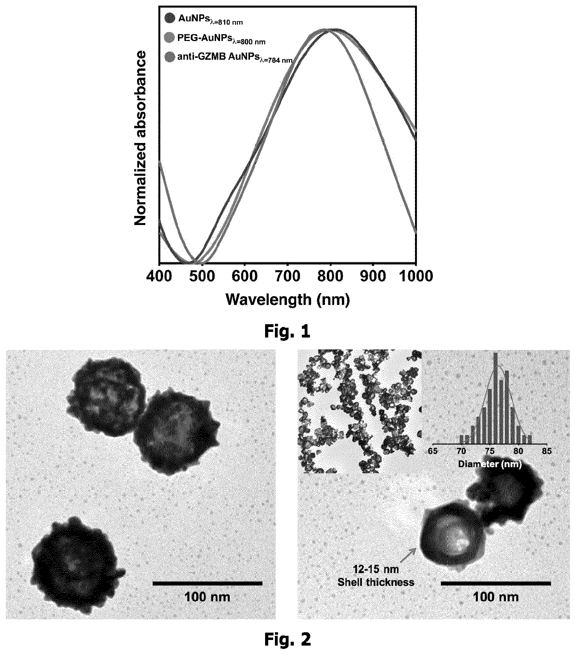

After the synthesis, the AuNPs, PEG-AuNPs, and anti-GZMB AuNPs were characterized by UV-vis spectroscopy. The absorption spectra obtained are shown in Figure 1 and it is possible to observe that the three systems exhibit resonance plasmons or absorption maxima around 800 nm, which is the common optical behavior of nanometric systems composed of gold, however, the modification of the surface with PEG on the surface of the AuNPs induces a shift of 10 nm towards the blue color, while the functionalization with anti-GZMB antibodies a shift of 26 nm; however, the resonance plasmon of the final system to be used to load NK cells remains within the near-infrared window, which is necessary to maintain its photothermal activity after being irradiated with infrared light.

**EXAMPLE 2**

In this example, the size, and Z-potential characteristics were determined, as well as the morphology of the AuNPs obtained in Example 1 in accordance with the principles of the present invention.

Table 1 shows the size and Z-potential determinations by dynamic light scattering (DLS) and laser Doppler electrophoresis, respectively, of the AuNPs, PEG-AuNPs, and anti-GZMB AuNPs. The determination of size, Z-potential and dispersity for the AuNPs was carried out in solutions with a nominal concentration of 5 $\mu$g/mL of gold in DTS1060 cells (Malvern Instruments Ltd). The equipment was adjusted to a temperature of 25 °C, an equilibrium time between readings of 120 s, a refractive index of 1.332, and an absorption of 0.080 using deionized water as a dispersing medium with a viscosity of 0.8872 cP in all readings. As seen in Table 1, the modification of the surface of AuNPs with PEG and subsequent functionalization with monoclonal antibodies induced significant changes in the surface charge of the AuNPs, since the addition of PEG to the surface inverted the net surface charge of the nanoparticles from -35.4 mV to 30.5 mV, which is consistent with the net positive charge of the -NH$_2$ ends of the PEG used; regarding the addition of the monoclonal antibody, the charge went from 30.5 mV to 24.5 mV, which can be explained by a partial coating of the surface with antibodies and the characteristic negative charge of IgG isotype antibodies. Regarding the size, it is possible to observe that the AuNPs exhibit gradual increases in their hydrodynamic diameters after the addition of PEG and anti-GZMB antibodies on their surface, however, this size is a virtual measure, which depends mainly on the dispersing medium, for which is necessary to corroborate the size obtained by transmission electron microscopy. The size, Z-potential, and dispersity data of the AuNPs obtained in Example 1 are shown in the following table:

**TABLE 1**

|  | Hydrodynamic diameter (nm) | Z-potential (mV) | Dispersity |
|---|---|---|---|
| **AuNPs** | 81.47 ± 2.87 | -35.4 ± 0.96 | 0.185 ± 0.09 |
| **PEG-AuNPs** | 88.54 ± 0.84 | +30.5 ± 1.01 | 0.121 ± 0.021 |
| **anti-GZMB AuNPs** | 99.45 ± 5.97 | +24.5 ± 0.86 | 0.110 ± 1.01 |
| The data are shown as mean ± DE; *n*=3. | | | |

After determining the sizes obtained by DLS, these nanoparticles were visualized through transmission electron microscopy (TEM) in a Morgagni M-268 microscope (FEI™) operated at 180 kV; AuNPs samples subjected to TEM visualization were deposited on copper grids coated with Formvar® (PELCO ® - 01700-F) depositing 5 $\mu$L of the AuNPs suspension containing 40 $\mu$g/mL of gold in deionized water, and was allowed to dry for 4 hours at room temperature in a desiccator; due to the optical and conductive properties of gold, this grid did not require additional treatment for visualization. The analysis of the distribution and histogram of size frequencies was performed with ImageJ2 software (NIH). Figure 2 shows that the AuNPs synthesized in Example 1 have a spheroid morphology, rough surfaces, and a shell thickness of between 12 and 15 nm. After visualization, an analysis of the size distribution was performed, observing that the AuNPs have real sizes mostly between 70 and 80 nm, which is consistent with the findings obtained by DLS shown in Table 1; it is worth mentioning that the modification by PEG and the addition of antibodies to the surface of the AuNPs cannot be visualized through TEM.

**EXAMPLE 3**

In this example, was evaluated the ability of the AuNPs obtained in Example 1 to transform light energy into heat after being irradiated with an infrared laser with a wavelength of 800 nm, which is used in this invention as a source of electromagnetic radiation.

The heating profile and photothermal stability of anti-GZMB AuNPs were evaluated by continuous irradiation of AuNPs suspensions (1800 μL, containing 40 μg/mL of gold in deionized water) for 180 min; for this experiment, the laser was turned on for 30 min until a plateau in temperature was observed, and after this, the laser was turned off for 15 min to cool the solution, repeating this procedure four times; for this assay, the anti-GZMB AuNPs were kept under constant magnetic stirring, and were kept in quartz cells with a lid (JASCO - 0553-FLOUR), and the irradiation was performed with an optical fiber directed at the wall of the quartz cell (area of the laser beam: 0.031 cm$^2$, 10.5 W/cm$^2$). The system temperature was monitored every minute using a *k*-type thermocouple (Amprobe - TMD-51) immersed in the suspension. The heating and cooling curve was constructed by plotting $\Delta T$ vs. time in minutes.

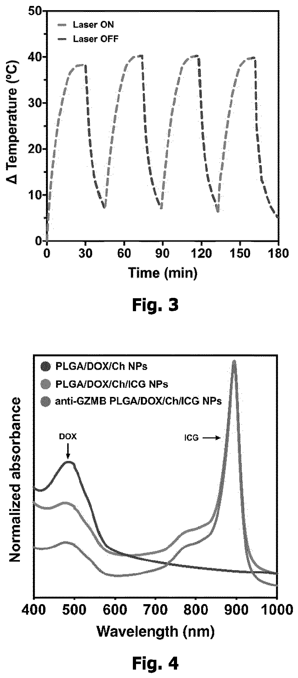

As shown in Figure 3, the irradiation of AuNPs for 30 min induces an oscillating temperature increase of 35 °C over the initial temperature, and after cooling, this phenomenon is replicated in serial irradiations to the system, which indicates that our system has photothermal stability. With the above, it is verified that AuNPs can efficiently convert light energy into heat and increase the temperature spatio-temporally on demand, which can be used to induce necrosis by hyperthermia in tumor cells. For purposes of this invention, only AuNPs with resonance plasmons located at 800 ± 15 nm will be used in order to favor the plasmonic effect provided by the optics of our laser ($\lambda$=800nm).

## EXAMPLE 4

The method for the preparation of biocompatible stimulated-release photoresponsive nanoparticles is described, based on biocompatible polymeric nanoparticles loaded with doxorubicin (DOX) with spatio-temporally activatable photothermal activity through infrared radiation, modification and inversion of the surface's potential after chitosan coating, subsequent indocyanine green (ICG) photosensitizing agent physisorption, and functionalization with anti-GZMB monoclonal antibodies according to one embodiment of the present invention.

The synthesis of Poly Lactic-co-Glycolic Acid (PLGA) nanoparticles containing doxorubicin (PLGA/DOX), coated with chitosan (PLGA/DOX/Ch), with ICG physisorbed on its surface (PLGA/DOX/Ch/ICG) was carried out through the nanoprecipitation method, which is indicated to encapsulate insoluble drugs, thus, the insoluble weak base of DOX was used for the synthesis process. The synthesis of PLGA/DOX NPs was carried out by dissolving 15 mg of the PLGA P.M. 5000-10000 Da (PolySciTech$^®$ - AP081) and 2.0 mg of DOX in 850 μL of reagent grade acetone, 100 μL of absolute ethanol and 50 μL of anhydrous methanol. Subsequently, the PLGA/DOX solution was added dropwise to 15 mL of a 1.0% (w/v) PVA aqueous solution, maintaining vigorous magnetic stirring during the dripping process. After incorporating the inorganic phase into the aqueous phase, the suspension was kept under vigorous magnetic stirring for 4 h to evaporate the solvents; After evaporation, the PLGA/DOX NPs were washed twice with deionized water by centrifugation at 10,000 *g*, and the surface was modified with chitosan. This coating induces inversion of the surface charge of the PLGA/DOX NPs by providing terminal -NH$_2$ ends that favor the electrostatic physisorption of ICG; the coating process is favored because our synthesis method gives rise to PLGA/DOX NPs with a net negative charge on their surface. After the synthesis and washing of PLGA/DOX NPs, 15 mg of PLGA/DOX NPs were resuspended in 15 mL of deionized water and were added 3 mg of low molecular weight chitosan (Sigma Aldrich - 448869), dissolved in 150 μL of acetic acid acid at 1%; the coating process was carried out by constant magnetic stirring for 8 hours. After this time, the PLGA/DOX/Ch NPs were washed twice with deionized water by centrifugation at 10,000 *g,* resuspended in 10 mL of deionized water, 200 μg of ICG were added, and the suspension was kept under constant magnetic stirring for 12 h to favor ICG physisorption on the surface of PLGA/DOX/Ch NPs. After this time, a wash was performed by centrifugation at 10,000 *g* to remove excess of ICG, the NPs were resuspended in PBS and the surface was functionalized with anti-GZMB monoclonal antibodies, with a range of 1 to 20 antibodies per nanoparticle. The functionalization was carried out through microfluidics and covalent entanglement between the -NH$_2$ terminal ends of the chitosan and the -COOH end of the Fc fraction of the monoclonal antibody. Prior to cross-linking, the monoclonal antibody was pre-activated with EDC and sulfo-NHS in MES buffer (Sigma-Aldrich - M8250) at a pH 6.0 to form amino-reactive ends in the -COOH region of the antibody to subsequently covalently bind them to the terminal -NH$_2$ ends present on the surface of the nanoparticle; this was achieved through the use of a microchip that allowed the controlled interaction of the monoclonal antibody with the nanoparticles in a laminar interface. Functionalization with anti-GZMB monoclonal antibodies in each nanoparticle was confirmed using the NanoOrange$^®$ protein quantification kit (Invitrogen$^{™}$ - N666), with a range of 1 to 20 antibodies per nanoparticle.

After the synthesis, the systems conformed of PLGA/DOX/Ch, PLGA/DOX/Ch/ICG, and anti-GZMB PLGA/DOX/Ch/ICG were characterized by means of UV-vis spectroscopy. The absorption spectra obtained are shown in Figure 4 and it is possible to observe that the PLGA/DOX/Ch NPs exhibit an absorption region between 450 and 550 nm that corresponds to the internalized DOX in the nanoparticles; regarding PLGA/DOX/Ch/ICG and anti-GZMB PLGA/DOX/ICG NPs, a characteristic peak is observed between 850 and 910 nm that allows photothermal and photodynamic activity to be provided to the system after being irradiated with infrared light; this photothermal activity induced by the excitation of the ICG photosensitizing agent also allows the polymeric matrix to be degraded and the drug DOX encapsulated in the nanoparticle to be released, allowing the establishment of a multimodal cytotoxic therapy that can be activated spatio-temporally with infrared radiation in tumor cells.

## EXAMPLE 5

In this example, the size and Z-potential characteristics, as well as the morphology of PLGA NPs obtained in Example 4 were determined in accordance with the principles of the present invention.

Table 2 shows the determinations of size and Z-potential by dynamic light scattering (DLS) and Doppler laser electrophoresis, respectively, of PLGA/DOX/Ch, PLGA/DOX/Ch/ICG and anti-GMZB PLGA/DOX/Ch/ICG NPs. The determination of size, Z-potential and dispersity for the PLGA NPs was carried out in solutions with a nominal concentration of 0.5 mg/mL of PLGA in DTS1060 cells (Malvern Instruments Ltd). The equipment was adjusted to a temperature of 25 °C, an equilibrium time between readings of 120 s, a refractive index of 1.460 and an absorption of 0.020 using deionized water as a dispersing medium with a viscosity of 0.8872 cP in all readings. As shown in Table 2, the PLGA/DOX NPs initially have a net negative charge that, after functionalization with Chitosan, goes from -40.7 to +60.4 mV, indicating that the chitosan cationic polysaccharide and the $-NH_2$ ends that constitute it, manage to reverse the surface potential of PLGA/DOX NPs. After surface modification, ICG (negatively charged) was physisorbed on the surface of PLGA/DOX/Ch NPs where it is clear that after physisorption, the charge of the system became slightly more negative. Finally, the conjugation of the anti-GZMB antibody was performed on the surface of the PLGA/DOX/Ch/ICG NPs, where, like the data shown in Table 1, the functionalization of the system with the antibody became slightly more negative, and with a similar magnitude of change in potential than that observed in PEG-AuNPs; in this case, the potential went from +31.9 to +16.1 mV, which confirms that anti-GZMB was added to the surface of the system. Regarding the size, it is possible to observe that the PLGA NPs exhibit gradual increases in their hydrodynamic diameters after the addition of chitosan, ICG and anti-GZMB antibodies on their surface, however this size is a virtual measure which depends mainly on the dispersant medium, for which it is necessary to corroborate the size obtained by scanning electron microscopy and atomic force microscopy. The size, Z-potential and dispersity data of the PLGA NPs obtained in Example 4 are shown in the following table:

**TABLE 2**

|  | Hydrodynamic diameter (nm) | Z-potential (mV) | Dispersity |
|---|---|---|---|
| **PLGA/DOX NPs** | 72.3 ± 2.89 | -40.7 ± 3.70 | 0.090 ± 0.010 |
| **PLGA/DOX/Ch NPs** | 87.6 ± 2.11 | +60.4 ± 4.78 | 0.119 ± 0.023 |
| **PLGA/DOX/Ch/ICG NPs** | 92.8 ± 1.61 | +31.9 ± 3.44 | 0.123 ± 0.012 |
| **Anti-GZMB PLGA/DOX/Ch/ICG NPs** | 101.2 ± 2.01 | +16.1 ± 1.09 | 0.156 ± 0.031 |
| The data are shown as mean ± DE; *n*=3. | | | |

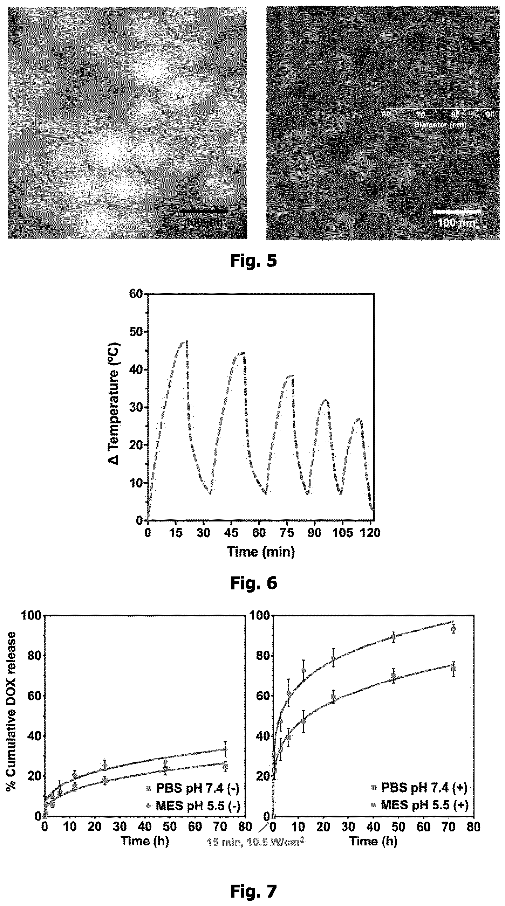

After determining the sizes obtained by DLS, these nanoparticles were visualized through Scanning Electron Microscopy (SEM) and Atomic Force Microscopy (AFM). Scanning electron microscopy was carried out in a MIRA3 microscope (TESCAN) using a voltage of 30.0 kV; the PLGA NPs samples subjected to SEM analysis were prepared by depositing 30 μL of a suspension containing 2 mg/mL of PLGA on a 10 × 10 mm silicon chip with a thickness between 475 - 575 μm with a resistance of 1- 30 Ohms (PELSCO ® - 16010) and subsequently, the chip was placed in a desiccator for 48 h and after drying, the chip was covered with gold for 15 seconds in a Sputter Coater (Balzers SCD 50), and visualization of the samples was carried out. Regarding the samples subjected to visualization by AFM (JEOL JSPM-4210). The analysis of the distribution and histogram of size frequencies was performed with ImageJ2 software (NIH) from the images obtained by SEM. Figure 5 shows the micrographs obtained by AFM (left) and SEM (right) and in both images it is possible to see that the PLGA NPs obtained have sizes smaller than 100 nm; regarding the size distribution analysis, most sizes range between 70 and 90 nm, which are consistent with the results obtained by DLS shown in Table 2.

Together, the results of size and Z-potential of PLGA NPs in addition to those shown in Table 1 and Figure 2 belonging to AuNPs, show that both types of nanoparticles have sizes around 80 nm and, besides, both have a net positive charge; this is necessary to facilitate its interaction with plasma membranes, primarily through electrostatic attraction in regions rich in cholesterol.

## EXAMPLE 6

In this example, was evaluated the ability of PLGA NPs obtained in Example 4 to transform light energy into heat after being irradiated with an infrared laser with a resonance wavelength of 800 nm, which is used in this invention as source of electromagnetic radiation.

The heating profile and photothermal stability of anti-GZMB PLGA/DOX/Ch/ICG NPs was evaluated by irradiating suspensions of PLGA NPs (1800 μL, containing 5 mg/mL of PLGA in deionized water) for 120 min; for this experiment, the laser was turned on for 20 min until a plateau in temperature was observed, and after this, the laser was turned off for 15 min to cool the solution; for this assay, the anti-GZMB PLGA/DOX/Ch/ICG NPs were kept under constant magnetic stirring in quartz cells with a lid (JASCO - 0553-FLOUR) and irradiation was performed with optics directed at the cell wall (Laser beam area: 0.031 cm$^2$, 10.5 W/cm$^2$). The system temperature was monitored every minute using a k-type thermocouple (Amprobe - TMD-51) immersed in the suspension. The heating and cooling curve was constructed by plotting $\Delta$T vs. time in minutes.

As shown in Figure 6, the irradiation of PLGA nanoparticles in an aqueous medium for 20 min induced a temperature increase of 45 °C over the initial temperature and after cooling, the temperature increase in each irradiation cycle gradually decreased, which can be explained from the photodegradation of the photosensitizing agent ICG. The foregoing demonstrates that the anti-GZMB PLGA/DOX/Ch/ICG NPs can efficiently convert light energy into heat and increase the spatio-temporal temperature on demand, and although it is observed that the photostability of the system decreases with each cycle of irradiation, the maximum temperature increase reached in the fifth irradiation cycle is still accompanied by the release of DOX and, in a biological scenario, by the generation of reactive oxygen species (photodynamic effect), which allows these NPs to be used to establish a multimodal treatment to induce apoptosis and necrosis in tumor cells mediated by a chemotherapeutic, photothermal, and photodynamic effect.

## EXAMPLE 7

In this example, an assay was carried out to determine the release profile of DOX contained in anti-GZMB PLGA/DOX/Ch/ICG obtained in Example 4 in the presence and absence of infrared radiation from a laser with a wavelength resonance of 800 nm, which is used as the irradiation source in this invention.

To evaluate the DOX release profile contained in anti-GZMB PLGA/DOX/Ch/ICG NPs, firstly, the quantification of the encapsulation percentage in the nanoparticle was performed by dissolving the PLGA NPs pellet in acetone, quantifying the fluorescence intensity and interpolating fluorescence intensity against DOX standard; this assay was performed in fluorescence plates of 96-well Clear Bottom (Corning® - CLS3904) using excitation and emission at 488 and 560 nm, respectively. The results of the quantification, in weight percentage of DOX with respect to the mass of PLGA present in the nanoparticles, are shown in Table 3.

**TABLE 3**

|  | Encapsulation percentage (w/w) |
|---|---|
| **DOX** | 7.32 $\pm$ 1.4 |
| The data are shown as mean $\pm$ DE; n=3. | |

After quantification, the DOX release profile of anti-GZMB PLGA/DOX/Ch/ICG NPs was determined in the presence and absence of radiation with infrared light at 800 nm. For this assay, 5.0 mL of anti-GZMB PLGA/DOX/ICG NPs were used at a nominal concentration of 5 mg/mL of PLGA contained in 50 mL conical tubes. The DOX release kinetics was evaluated by dispersing the NPs in two buffers: in MES buffer pH 5.5 (simulating lysosomal pH) and in PBS buffer pH 7.4 (simulating physiological pH). The DOX release profile was performed at a temperature of 37 $\pm$ 2 °C for 72 h and throughout the release process, the suspensions were kept under constant magnetic stirring. DOX quantification was performed at 0.5, 1, 2, 4, 8, 12, 24, 48, and 72 h and DOX released quantification was performed by centrifuging the nanoparticles for 15 min at 10,000 $g$ and quantifying the amount of DOX present in the supernatant; t each quantification point, the supernatant was replaced with fresh buffer tempered at 37°C. In the working groups where the effect of irradiation with infrared light was evaluated, initially, the total mass of NPs was resuspended in a volume of 2.5 mL of buffer tempered at 37 °C and subsequently irradiated with a laser at 800 nm (15 min, 10.5 W/cm$^2$, t=0) in quartz cells with constant magnetic stirring. After irradiation, the nanoparticle suspensions were transferred to 50 mL conical tubes, the volume was completed at 5 mL and the release kinetics was started. The DOX release profile was reported as percentage of accumulated DOX versus time in hours.

As observed in Figure 7, the anti-GZMB PLGA/DOX/Ch/ICG NPs exhibit evident differences in their release profiles in the absence (-) and presence of irradiation with infrared light (+). Regarding the release profiles in the absence of irradiation at 72 h, at pH 5.5 a cumulative release of DOX of 30% is reached, while at pH 7.4 a cumulative release of DOX of 22% is obtained. Regarding the groups irradiated with infrared light, it is possible to observe that at 72 h, the suspension with pH 5.5 reached a DOX release greater than 90%, while with pH 7.4 a cumulative release of 70%. These results indicate that the anti-GZMB PLGA/DOX/Ch/ICG NPs, in the absence of irradiation, constitute a prolonged-release system, while, in the presence of infrared radiation, the system allows encapsulated DOX to be released quickly and on-demand, and together with the results obtained in Example 6, these results confirm that the anti-GZMB PLGA/DOX/Ch/ICG NPs represent a multimodal therapeutic system with spatio-temporally activatable photothermal activity through infrared irradiation.

**EXAMPLE 8**

In this example, an assay was carried out to estimate the effective dose 50 (ED$_{50}$) of anti-GZMB AuNPs and anti-GZMB PLGA/DOX/Ch/ICG NPs obtained in Examples 1 and 4, in triple negative breast cancer cells (HCC70) and HER2+ (HCC1954) breast cancer cells, in the absence and presence of infrared light irradiation according to two embodiments of the present invention.

To assess the above, 10,000 triple negative (HCC70) or HER2+ (HCC1954) breast cancer cells were cultured in 96-well culture plates using RPMI-1640 culture medium added with 10% fetal bovine serum (Gibco$^{©}$), streptomycin at 100 $\mu$g/mL and penicillin at 100 U/mL (Sigma-Aldrich$^{®}$ - P4333) for 24 hours. After 24 hours, the culture medium was replaced with 90 $\mu$L of fresh culture medium and the remaining volume to complete 100 $\mu$L was completed with anti-GZMB AuNPs or anti-GZMB PLGA/DOX/Ch/ICG NPs dissolved in culture medium. The dosage of AuNPs in this assay was carried out from the nominal concentration of gold present in the suspension and the following concentrations were evaluated: 5.0, 10.0, 50.0, 100.0, 500.0, 1000.0 and 5000.0 $\mu$M, while for the PLGA NPs , the dosage was made from the nominal concentration of DOX and the concentrations evaluated were 5.0, 10.0, 50.0, 100.0, 500.0, 1000.0 and 5000.0 nM. After the incorporation of the metallic or polymeric nanoparticles to the problem wells, the cells were incubated for 24 h. After the incubation time, the wells with non-irradiated (-) cells were washed once with PBS and the culture medium was replaced with 90 $\mu$L of PBS and 10 $\mu$L of Triton X-100 (Sigma-Aldrich$^{®}$) and viability was determined; regarding the experimental groups subjected to irradiation, the culture medium was replaced with 100 $\mu$L of fresh culture medium and the cells were irradiated with a resonance wavelength laser at 800 nm, using an optical fiber to irradiate only the bottom of the well (5 min, 6.25 W/cm$^2$). After irradiation, the wells with irradiated (+) cells were washed once with PBS, the well volume was replaced with 90 $\mu$L of PBS and 10 $\mu$L of Triton X-100, and viability was determined. Viability was determined through the total concentration of Lactate Dehydrogenase (LDH) with the Pierce$^{™}$ LDH cytotoxicity kit (Thermo Scientific), establishing the activity of LDH cells in untreated control wells as 100% viability. Finally, the ED$_{50}$ was estimated from a 4PL sigmoidal nonlinear fit of the viability percentage and Log of the concentration. Data are shown as mean $\pm$ standard deviation, n=6.

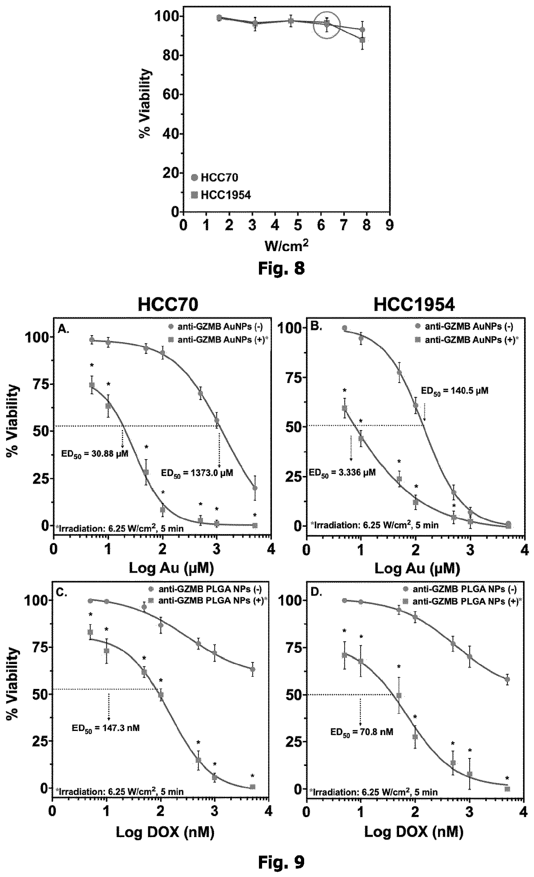

Before estimating the ED$_{50}$ of AuNPs and PLGA NPs, an assay was performed to evaluate the cytotoxic activity derived from irradiation with infrared light at 800 nm on triple negative (HCC70) and HER2+ (HCC1954) breast cancer cells at different irradiation intensities; the assay was performed under the same conditions as the previously described experiment, in the absence of both nanoparticles, evaluating the following irradiation intensities for 10 min: 1.5625, 3.125, 4.6875, 6.25, 7.8125 W/cm$^2$. The results for the effects of irradiation with infrared light are shown in Figure 8 and as can be seen, irradiation with infrared light has no significant effect on cell viability of HCC70 or HCC1954 cells at doses up to 7.8125 W/cm$^2$ (Mann-Whitney U test, p>0.9999, n=3), however, for purposes of this invention, an irradiation intensity equal to or less than 6.25 W/cm$^2$ (red circle) will be used.

Regarding the estimation of the ED$_{50}$ of AuNPs and PLGA NPs in the presence and absence of irradiation with infrared light in the cell lines HCC70 and HCC1954, the results in Figure 9 show that, for both types of nanoparticles and in all doses evaluated, the cytotoxic effect of NPs increases significantly when irradiated with infrared light (+) when compared to non-irradiated treatments (-) (Two-way ANOVA with Bonferroni *post-hoc* test; (*) p>0.05 with respect to non-irradiated group (-)). Regarding the groups treated with anti-GZMB AuNPs (Figures 9 Aand B), we have that HCC70 cells are 10 times more resistant to treatment with these nanoparticles compared to HCC1954 cells in the presence and absence of infrared light irradiation (1373.0 vs. 140.5 $\mu$M and 30.88 vs. 3.33 $\mu$M, respectively), and also, in both cell lines irradiation with infrared light in this treatment increases more than 40 times the cytotoxic activity when compared to its non-irradiated counterpart, which confirms that AuNPs constitute a photothermal system capable of inducing spatio-temporal cytotoxic activity when irradiated with infrared light at 800 nm. In the case of the anti-GZMB PLGA/DOX/Ch/ICG NPs (Figures 9 C and D), we have that this type of nanoparticles do not intrinsically have a considerable cytotoxic effect at 24 h, for which it was impossible to determine the ED$_{50}$ for both cell lines in the non-irradiated treatments (-) and, together with the results obtained in Figure 7, these results confirm that the PLGA NPs behave as a prolonged-release system. Regarding the cytotoxic effect of treatments irradiated with infrared light (+), it is possible to observe that HCC70 cells are less susceptible to multimodal therapy offered by PLGA NPs compared to HCC1954 cells (147.3 vs 70.8 nM). This, together with the results observed in Figures 9 A and B, can be explained by the intrinsic phenotypic differences between triple negative breast cancer (HCC70) and HER2+ (HCC1954) and the ability to mitigate oxidative stress induced by hyperthermia and some associated chemotherapeutic resistance mechanisms. Taken together, the results shown in Figure 9 indicate that the anti-GZMB AuNPs and PLGA/DOX/Ch/ICG NPs possess cytotoxic activity against tumor cells spatio-temporally activatable through infrared radiation.

**EXAMPLE 9**

In this example, an assay was carried out to evaluate the increase in the production of superoxide anion ($O_2^{•-}$) and singlet oxygen ($^1O_2$) from treatment with anti-GZMB AuNPs and anti-GZMB PLGA/DOX/Ch/ICG NPs obtained in Examples 1 and 4, in triple negative breast cancer cells (HCC70) and HER2+ (HCC1954) breast cancer cells in the absence and presence of infrared light irradiation according to two modalities of the present invention.

To assess the above, 10,000 triple negative (HCC70) or HER2+ (HCC1954) breast cancer cells were cultured in 96-well clear-bottom fluorescence culture plates (Corning® - CLS3904) using RPMI-1640 culture medium without phenol red (Gibco®) added with 10% fetal bovine serum (Gibco©), streptomycin at 100 $\mu$g/mL and penicillin at 100 U/mL (Sigma-Aldrich® - P4333) and pre-incubated for 24 hours. After the pre-incubation, at the non-irradiated (-) problem groups, the culture medium was replaced by 80 $\mu$L of fresh culture medium and 10 $\mu$L of anti-GZMB AuNPs or anti-GZMB PLGA/DOX/Ch/ICG NPs dissolved in culture medium. For this assay, the dosage of AuNPs was carried out from the nominal concentration of gold (Au) and the following concentrations were evaluated: 5.0, 50.0 and 500.0 $\mu$M, while for the PLGA NPs, the dosage was carried out from the nominal concentration of DOX and the concentrations evaluated were 10.0, 100.0 and 1000.0 nM, which are equivalent to a nominal concentration of 22.2, 222.2 and 2222.2 nM of ICG. After the incorporation of the metallic or polymeric nanoparticles to the problem wells, the wells were added with 10 $\mu$L of a solution containing 100 $\mu$g of NBT (Sigma-Aldrich® - N6876) dissolved in PBS or 10 $\mu$L of a solution stock 100 $\mu$M of Singlet Oxygen Sensor Green (Invitrogen™) dissolved in PBS and the cells were incubated for 24 h, protecting the plate from light throughout the procedure. Regarding the experimental groups subjected to irradiation (+), after 24 hours of pre-incubation, the culture medium was replaced with 90 $\mu$L of fresh culture medium and 10 $\mu$L of anti-GZMB AuNPs or anti-GZMB PLGA/DOX/Ch/ICG NPs dissolved in culture medium and cells were incubated for 24 hours. After the incubation time, the wells were washed twice with PBS, 90 $\mu$L of PBS were added and the wells were added with 10 $\mu$L of a solution containing 100 $\mu$g of NBT (Sigma-Aldrich® - N6876) dissolved in PBS or 10 $\mu$L of a 100 $\mu$M stock solution of Singlet Oxygen Sensor Green (Invitrogen™) dissolved in PBS. After the addition of NBT or Singlet Oxygen Sensor Green, the cells were irradiated with a resonance wavelength laser at 800 nm, using a fiber optic collimator to irradiate only the bottom of the well (5 min, 6.25 W/cm$^2$). After irradiation, the production of superoxide anion and singlet oxygen was determined. The production of superoxide anion ($O_2^{•-}$) was evaluated from the quantification of formazan, an insoluble product generated after the oxidation of NBT at the intracellular level by $O_2^{•-}$, for this, the culture medium was removed from the wells added with NBT, and were added 50 $\mu$L of 2 M KOH to saponify the cell membranes and after 5 minutes, 60 $\mu$L of DMSO were added to dissolve the formazan generated after treatment with NPs. The wells were read in a microplate reader at 620 nm and the absorbance obtained was corrected with the absorbance values obtained in control wells without cells. Regarding the production of singlet oxygen ($^1O_2$), this was determined from the intensity of fluorescence generated after complexation of $^1O_2$ with the selective probe Singlet Oxygen Sensor Green; the wells added with Singlet Oxygen Sensor Green were read in a microplate reader in fluorescence mode exciting at 488 nm and quantifying the fluorescence intensity at 525 nm; the fluorescence intensity generated in the non-irradiated treatments (-) was corrected with the fluorescence generated in control wells in the absence of cells. The production of superoxide anion and singlet oxygen was reported as an increase in production (number of times) concerning control wells without nanoparticles. Data are shown as mean $\pm$ standard deviation, n=6.

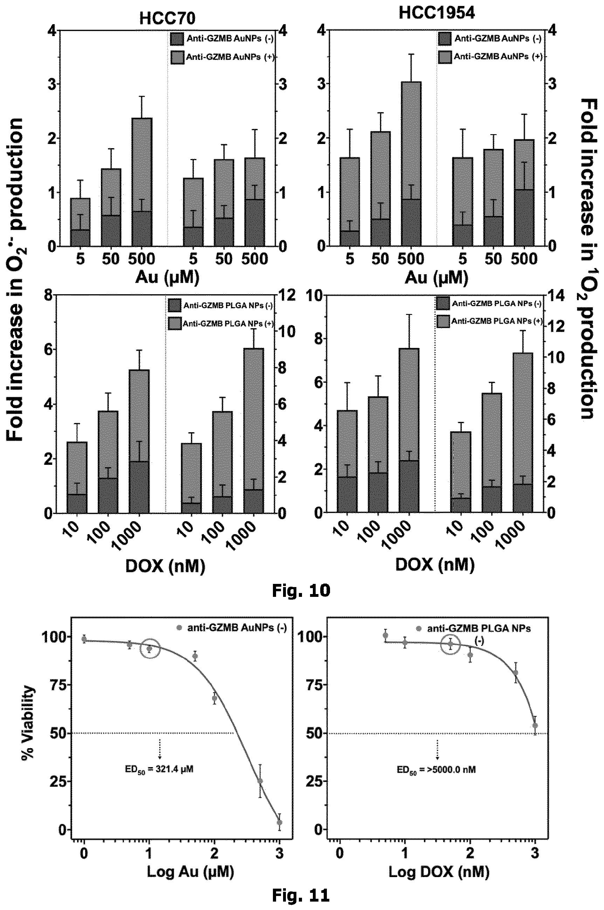

The increase in the production of superoxide anion ($O_2^{•-}$) and singlet oxygen ($^1O_2$) derived from treatment with anti-GZMB AuNPs and anti-GZMB PLGA/DOX/Ch/ICG NPs in the absence (-) and presence (+) of infrared light irradiation on triple negative (HCC70) and HER2+ (HCC1954) breast cancer cells is shown in Figure 10. In both treatments and in the absence of irradiation with infrared light, it can be observed that there is no significant increase in the production of superoxide anion and singlet oxygen, however, it is evident that irradiation with infrared light induces a dose-dependent increase in the production of both species, being more pronounced in the treatments with PLGA/DOX/Ch/ICG NPs. Regarding the treatment with anti-GZMB AuNPs shown in Figures 10 A and B, for both cell types and with the three doses evaluated, the production of superoxide anion in the irradiated treatments (+) follows a dose-dependent trend being slightly higher in HCC1954 cells, which is consistent with the susceptibility shown by this cell line to photothermal therapy for this type of nanoparticles concerning HCC70 cells shown in Figure 9; regarding the singlet oxygen production, in both cell types and in the presence of irradiation, the production of this species does not exhibit dose-dependent increases comparable to the production of superoxide anion, suggesting that treatment with anti-GZMB AuNPs is not accompanied by singlet oxygen production, since the production at doses of 50 and 500 $\mu$M in both cell types showed practically equal values. In relation to the treatment with anti-GZMB PLGA/DOX/Ch/ICG NPs shown in Figures 10 C and D, it is found that for both types of cells and in all the doses evaluated, there is a dose-dependent increase in the production of both species. Unlike anti-GZMB AuNPs, treatment with anti-GZMB PLGA/DOX/Ch/ICG NPs and irradiation with infrared light induces a dose-dependent increase in the production of both types of species in both types of cells, and with all the doses evaluated; in both types of cells, it is possible to observe that the production of superoxide anion is practically double compared to the treatment with AuNPs, while the production of singlet oxygen exhibits an evident dose-dependent increase significantly greater in both types of cells, which it is due to the *in situ* production of singlet oxygen by ICG after irradiation with infrared light. Taken together, the results shown in Figures 9 and 10 demonstrate that anti-GZMB AuNPs and anti-GZMB PLGA/DOX/Ch/ICG NPs are capable of inducing oxidative stress in cells from the on-demand production of reactive species of oxygen in cells after irradiation with infrared light, being the anti-GZMB PLGA/DOX/Ch/ICG NPs a system with higher oxidative activity due to the presence of photodynamic agent ICG. This demonstrates that cell death induced by both types of nanoparticles is accompanied by the production of reactive oxygen species and that their multimodal cytotoxic effect is spatio-temporally activatable through infrared light.

**EXAMPLE 10**

In this example, the effects on the viability of anti-GZMB AuNPs and anti-GZMB PLGA/DOX/Ch/ICG NPs obtained in Examples 1 and 4 in NK cells of the NKL cell line were evaluated, and were also chosen the doses to carry out the loading of both types of nanoparticles in NKL cells according to two embodiments of the present invention.

To assess the above, 10,000 NK cells of the NKL cell line were cultured in 96-well culture plates in 90 $\mu$L of RPMI-1640 culture medium added with 15% fetal bovine serum (Gibco©), streptomycin at 100 $\mu$g/mL, and penicillin at 100 U/mL (Sigma-Aldrich® - P4333), recombinant human interleukin 2 (IL-2) at 20 ng/mL (Biolegend® - 589104) and recombinant human interleukin 15 (IL-15) at 20 ng/mL (Biolegend® - 570304), and the remaining volume to complete 100 $\mu$L was completed with anti-GZMB AuNPs or anti-GZMB PLGA/DOX/Ch/ICG NPs dissolved in culture medium. The dosage of AuNPs in this assay was carried out from the nominal concentration of gold present in the suspension of nanoparticles and the following concentrations were evaluated: 1.0, 5.0, 10.0, 50.0, 100.0, 500.0, and 100.0 $\mu$M, while for the PLGA NPs, the dosage was made from the nominal concentration of DOX and the concentrations evaluated were 5.0, 10.0, 50.0, 100.0, 500.0, and 1000.0 nM. After the incorporation of the metallic or polymeric nanoparticles to the problem wells, the cells were incubated for 24 h. After the incubation time, the culture plates were centrifuged for 10 min at 1,000 $g$, the culture medium was carefully removed, the volume was replaced with 90 $\mu$L of PBS and 10 $\mu$L of Triton X-100 (Sigma-Aldrich®) and viability was determined. Viability was determined through the total concentration of Lactate Dehydrogenase (LDH) with the Pierce™ LDH cytotoxicity kit (Thermo Scientific), establishing the activity of LDH cells in untreated control wells as 100% viability. Finally, dose-response curves were constructed and the $ED_{50}$ was estimated from a 4PL sigmoidal nonlinear fit of the viability percentage and Log of the concentration. Data are shown as mean $\pm$ standard deviation, n=6. The effects on viability of anti-GZMB AuNPs and anti-GZMB PLGA NPs are shown in Figure 11.

The dose-response curves at 24 h shown in Figures 11 A and B indicate that natively anti-GZMB AuNPs have a higher cytotoxic effect than anti-GZMB PLGA NPs in NKL cells, which is consistent with the dose-response curves obtained in Figures 9 C and D where in both cases, it was impossible to determine the $ED_{50}$ in the absence of irradiation in the treatments with PLGA NPs. Regarding the dosage, it is found that the co-culture of NKL cells with concentrations greater than 10.0 $\mu$M of anti-GZMB AuNPs or 50.0 nM of anti-GZMB PLGA NPs induce a decrease in cell viability of around 15%, therefore, in this invention, the loading of nanoparticles in NKL cells will be carried out by co-cultivating at a dose of 10.0 $\mu$M of anti-GZMB AuNPs and 50.0 nM of anti-GZMB PLGA NPs (indicated in red circles) in order to avoid significant effects in the cell viability of NKL cells.

**EXAMPLE 11**

In this example, was carried out the loading of anti-GZMB AuNPs and anti-GZMB PLGA/DOX/Ch/ICG NPs obtained in Examples 1 and 4 in NKL cells under standard culture conditions, and the internalization of both types of nanoparticles was verified through multiphoton confocal microscopy and quantification in CD63+ secretory lysosomes according to two embodiments of the present invention.

Loading of anti-GZMB AuNPs and anti-GZMB PLGA/DOX/Ch/ICG NPs was performed by culturing NKL cells in 25 cm2 culture bottles (Nunc®) at a density of 200,000 cells/mL in RPMI-1640 culture medium added with 1% fetal bovine serum (Gibco©), streptomycin at 100 $\mu$g/mL and penicillin at 100 U/mL (Sigma-Aldrich® - P4333), recombinant human interleukin 2 (IL-2) at 20 ng/ mL (Biolegend® - 589104) and recombinant human interleukin 15 (IL-15) at 20 ng/mL (Biolegend® - 570304) for 12 hours. After 24 hours, the cells were centrifuged and the culture medium was replaced with fresh culture medium added with 15% fetal bovine serum containing 10.0 $\mu$M of anti-GZMB AuNPs or 50.0 nM of anti-GZMB PLGA NPs and the cells were cultured at a density of 250,000 cells/mL for 24 hours; after the addition of NPs, the cells were resuspended every 4 hours to favor the internalization of both types of nanoparticles. The bottles added with PLGA NPs were protected from light throughout the process to avoid photoactivation of ICG. After 24 hours of incubation, the cells were washed once with fresh culture medium and the cells were visualized in the multiphoton confocal microscope or quantified the nanoparticles in CD63+ secretory lysosomes.

Visualization of NKL cells loaded with anti-GZMB AuNPs or anti-GZMB PLGA/DOX/Ch/ICG NPs was performed on fixed cells by staining the nucleus and the endomembrane system. To do the above, the cells were washed twice with PBS and subsequently, the cell pellet was resuspended in 1 mL of 4% $p$-formaldehyde pH 6.9 (Sigma-Aldrich® -

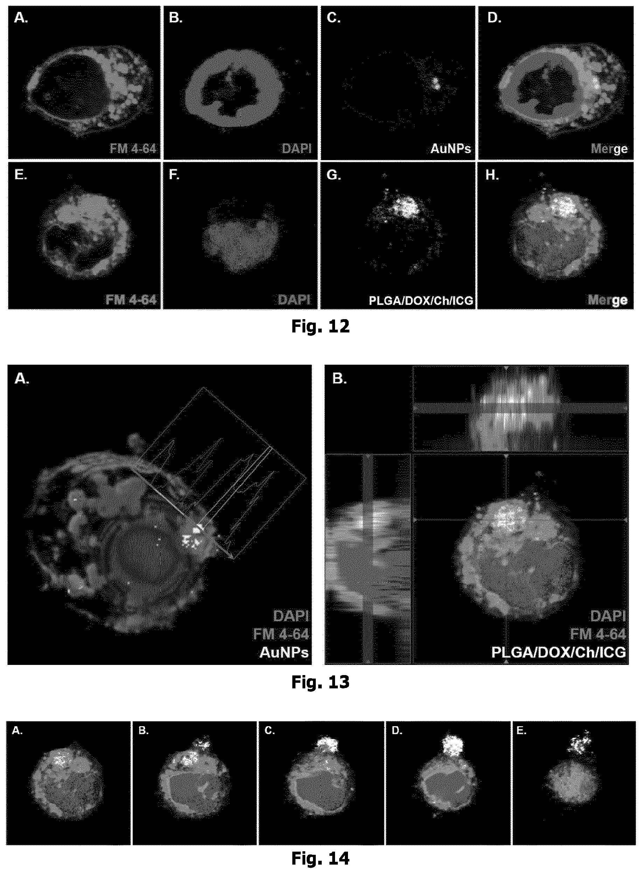

1.00496) and the cells were incubated in the dark for 30 min at room temperature. After 30 min, the cells were washed twice with PBS, resuspended in 100 μL of deionized water, and was added 1 μL of a solution containing 0.001 mg/mL of DAPI (Thermo Scientific™ - 62248) to stain the nucleus; after the addition of DAPI, the cells were incubated for 60 min at 4°C. After 60 min, the cells were washed twice with PBS, resuspended in 50 μL of deionized water and the endomembrane system was stained with FM 4-64 dye (Thermo Scientific™ - T13320) at a dilution of 1: 1000. After the addition of the FM 4-64 dye, the cells were incubated in the dark for 60 min and after the incubation time, the cells were washed twice with deionized water and the cells were mounted in a drop of agarose at 3% dissolved in deionized water for visualization in the Zeiss LSM880 NLO multiphoton confocal microscope. The images obtained are shown in Figures 12, 13 and 14, where it is possible to see the endomembrane system in red with the dye FM 4-64 (Exc/Em: 515/640 nm); the nucleus in blue contrasted with DAPI (Exc/Em: 358/461); AuNPs (Exc/Em: 750/560) in yellow and PLGA NPs in white (Exc/Em: 488/582).

Regarding the visualization of anti-GZMB AuNPs and anti-GZMB PLGA/DOX/Ch/ICG NPs, Figure 12 shows the internalization of both types of nanoparticles after incubation for 24 hours with the doses selected in Example 10. In these 1000X micrographs of NKL cells, it is possible to see that the AuNPs (Figures 12 A-D), although they can be internalized, these are internalized to a lesser extent than the PLGA NPs (Figures 12 E-H), which can be explained by the characteristics of friability and density of the material. After visualizing the NPs, their intracellular localization was specifically described with a spectrum of co-localization and axial Z localization as shown in Figure 13. In the case of AuNPs (Figure 13A), the co-localization spectrum shows that these NPs are specifically located in the endomembrane system in regions close to the nucleus, which suggests that these nanoparticles after their endocytosis are distributed to the Golgi network and the reticulum system where they can conjugate with Granzyme B. Regarding the PLGA NPs, the axial location of these nanoparticles shown in Figure 13B indicates that these NPs, like the AuNPs, are located in the endomembrane system, however, their distribution covers regions apical to the nucleus, which it could suggest that these nanoparticles have already been distributed by the Golgi network and are already loaded inside the cytotoxic granules. For this reason, a Z-stack of NKL cells loaded with PLGA NPs was performed, which is shown in Figure 14. In this series of micrographs, it can be seen that the PLGA NPs are indeed located in lysosomes (A), however, after analyzing the lower planes (B-E) it can be seen that these PLGA NPs are being exocytosed, which suggests that the PLGA NPs are located in CD63+ secretory lysosomes and it also proves that NPs internalized by NKL cells can be mobilized intracellularly.

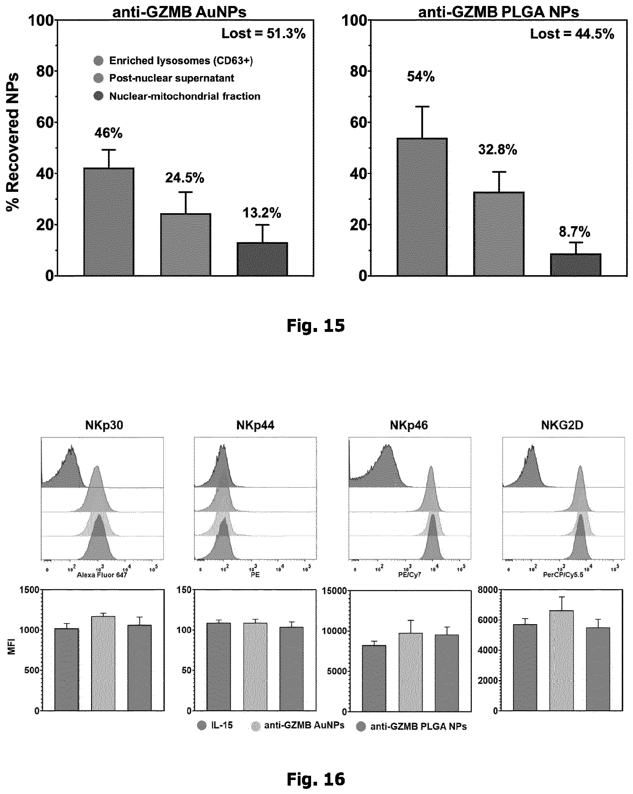

To corroborate the intracellular localization of anti-GZMB AuNPs and anti-GZMB PLGA/DOX/Ch/ICG NPs, we proceeded to quantify anti-GZMB AuNPs or anti-GZMB PLGA/DOX/Ch/ICG NPs in CD63+ secretory lysosomes, which was carried out from cell fractionation by centrifugation. As an initial step, NKL cells ($1 \times 10^8$) were washed twice with 5 mL of homogenization buffer containing 10 mM acetic acid, 1 mM EDTA, 190 mM sucrose, 10 mM triethanolamine, added with the cOmplete™ Protease Inhibitor Cocktail (Roche). After washing, the cell pellet was homogenized on ice using 3 mL of homogenization buffer by passing the cells 10 to 15 times in a glass cell homogenizer with a pistil, with a rose distance of 10 μm (Sigma Aldrich - D9063). After homogenization, the lysate was transferred to a 15 mL conical tube, the nuclear-mitochondrial fraction was separated by centrifugation (5,000*g* for 15 min at 4°C), and the post-nuclear supernatant was transferred to a 50 mL conical tube containing 10 mL of Percoll pH 8.5-9.5 (Sigma-Aldrich® - P1644). Subsequently, ultracentrifugation (15,000*g* for 120 min at 4°C) was carried out to obtain the lysosomal fraction (white band at the bottom of the tube) and it was carefully transferred to a 15 mL conical tube. After obtaining the lysosomal fraction, it was ultracentrifuged to obtain a pellet (15,000*g* for 240 min at 4°C), it was resuspended in a flatbottomed tube with 100 μL of exosome isolation buffer (Invitrogen™) and 20 μL of Dynabeads anti-CD63 (Invitrogen™ - 10606D). After the addition of anti-CD63 Dynabeads, the tube was kept under constant stirring with an orbital shaker for 12 hours at 4°C, and after 12 hours, the Dynabeads were transferred to Lo-Bind tubes (Eppendorf®) and washed twice with 500 μL of PBS using a DynaMag™-Spin. After washing, the Dynabeads were resuspended in 250 μL of PBS and the AuNPs or PLGA NPs contained in CD63+ lysosomes were quantified. The quantification of AuNPs in CD63+ enriched lysosomes, post-nuclear supernatant, and nuclear-mitochondrial fraction was performed by ICP/MS (Agilent 7700 series) from an acid digestion of the analysis matrix and subsequent gold quantification. Regarding the quantification of DOX, this was carried out by UPLC in tandem with MS/MS (ACQUITY™ - Waters Corp.) from the monitoring of the ionic transition (m/z) of DOX from 544.25 to 397.16. For both cases, it was confirmed that the recovery percentage was greater than 95% using gold standard curves ($HAuCl_4$) and hydrophobicized DOX, subjecting them to the same extraction and purification conditions as the problem samples. Data are reported as percent recovery and data are shown as mean ± standard deviation, n=3. The results of the quantification of anti-GZMB AuNPs and anti-GZMB PLGA/DOX/Ch/ICG NPs are shown in Figure 15.

As can be seen in Figure 15, both nanoparticles have a preferential location in the CD63+ lysosomal fraction, followed by a location in the post-nuclear supernatant and show a low distribution in the mitochondrial nuclear fraction. This indicates that NPs after their endocytosis are preferentially distributed in CD63+ secretory lysosomes but, in addition, the presence of NPs in the post-nuclear supernatant indicates that both types of nanoparticles can be located unspecifically in the cytoplasm but also, in some point of their intracellular traffic, they can be localized in a unspecific way in the nuclear and mitochondrial region; however, the above does not indicate that NPs are internalized in the nucleus or mitochondria since, due to size and signaling molecules, the entry into these organelles is virtually impossible.

Taken together, the results shown in Figures 12, 13, 14, and 15 indicate that anti-GMZB AuNPs and anti-GZMB PLGA/DOX/Ch/ICG NPs are capable of being internalized in NKL cells and preferentially distributed in CD63+ secretory lysosomes, which indicates that this type of nanoparticles have the ideal loading and size conditions to be loaded into NKL cells under standard culture conditions.

## EXAMPLE 12

In this example, was evaluated the loading effects of anti-GZMB AuNPs and anti-GZMB PLGA/DOX/Ch/ICG NPs obtained in Examples 1 and 4 on the expression of natural cytotoxicity receptors (NKp30, NKp44, NKp46, and NKG2D), cytokine production (TNF-$\alpha$ and IFNy) and degranulation against triple negative breast cancer cells (HCC70) and HER2+ (HCC1954) breast cancer cells by flow cytometry according to two modalities of the present invention.

To assess the loading effects of anti-GZMB AuNPs and anti-GZMB PLGA/DOX/Ch/ICG NPs on NKL cell natural cytotoxicity receptor expression, 250,000 NKL cells were cultured in 24-well plates in 1 mL RPMI-1640 culture medium added with 15% fetal bovine serum (Gibco©), streptomycin at 100 $\mu$g/mL, and penicillin at 100 U/mL (Sigma-Aldrich® - P4333), recombinant human interleukin 15 (IL-15) at 20 ng/mL (Biolegend®- 570304) adjusting the culture medium to a concentration of 10.0 $\mu$M of anti-GZMB AuNPs or 50.0 nM of anti-GZMB PLGA NPs, for 24 hours. After 24 hours, the cells were transferred to cytometry tubes and washed twice at 500g with staining buffer (Biolegend®) and finally resuspended in 100 $\mu$L of staining buffer to label with antibodies with the concentrations and conditions of temperature indicated by the manufacturer (Biolegend®). Antibodies used in this panel of analysis were Alexa Fluor® 647 anti-human CD337 (NKp30) clone P30-15, PE anti-human CD336 (NKp44) clone P44-8, PE/Cy7 anti-human CD335 (NKp46) clone 9E2 and PerCP/Cy5.5 anti-human CD314 (NKG2D) clone 1D11. After staining and washing off excess antibodies, the cells were analyzed in the FACSCanto II (BD ®) cytometer, performing a compensation using FMO, obtaining a minimum of 100,000 events per assay. Histograms of representative experiments and average mean fluorescence intensity are shown in Figure 16.

As seen in Figure 16, no significant differences in the expression of NKp30, NKp44, NKp46 or NKG2D are observed after loading anti-GZMB AuNPs and anti-GZMB PLGA/DOX/Ch/ICG NPs in NKL cells when compared to control cells (IL-15) without nanoparticles (Kruskal-Wallis test with Dunn's *post-hoc* test, n=6). This suggests that the loading of both types of NPs does not induce effects on their activating receptors and, therefore, on their recognition mechanisms for tumor cells.

Regarding the effects on the production of TNF-$\alpha$ and IFNy, this was evaluated in two conditions: in the absence and presence of activating stimuli. To do the above, 500,000 NKL cells were cultured in 24-well plates in 1 mL RPMI-1640 culture medium added with 15% fetal bovine serum (Gibco©), 100 $\mu$g/mL streptomycin, and 100 U/mL penicillin (Sigma-Aldrich® - P4333), human recombinant interleukin 15 (IL-15) at 20 ng/mL (Biolegend® - 570304) adjusting the culture medium to a concentration of 10.0 $\mu$M anti-GZMB AuNPs or 50.0 nM of anti-GZMB PLGA NPs; to evaluate the production of cytokines in the absence of activating stimuli, the cells were incubated for 6 hours with both types of nanoparticles, adding Brefeldin A (Biolegend®) at the concentrations indicated by the manufacturer after one hour of culture with NPs. After 6 hours, the cells were fixed with 4% p -formaldehyde for 20 min at room temperature, were washed twice with permeabilization buffer and intracellular staining of TNF-$\alpha$ and IFNy was performed according to the manufacturer's instructions (Biolegend®). Cytokine production in the presence of an activating stimulus was performed with NKL cells preloaded for 12 hours with both types of nanoparticles. To do this, 500,000 NKL cells preloaded with NPs were cultured in 1 mL RPMI-1640 culture medium added with 15% fetal bovine serum (Gibco©), streptomycin at 100 $\mu$g/mL, and penicillin at 100 U/mL ( Sigma-Aldrich® - P4333), human recombinant interleukin 15 (IL-15) at 20 ng/mL (Biolegend® - 570304) and activation cocktail (Biolegend®); after the addition of the activation cocktail, the cells were incubated for 6 hours, adding Brefeldin A (Biolegend®) at the concentrations indicated by the manufacturer after one hour of cultivation with the activation cocktail. After the incubation time, the cells were labeled intracellularly as previously described and were analyzed in the flow cytometer. The antibodies used in this panel were PE/Cy7 anti-human TNF-$\alpha$ clone MAb11 and PE anti-human IFN-$\gamma$ clone B27.

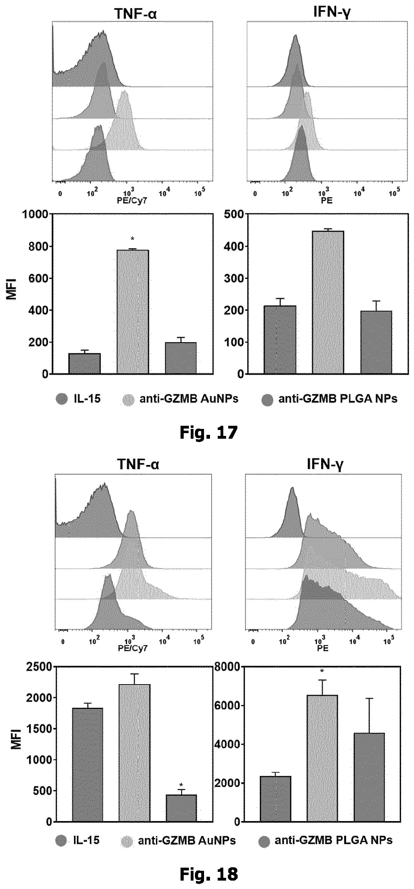

The results obtained are shown in Figures 17 and 18, where the production of cytokines is shown in the absence and presence of an activating stimulus, respectively.

As seen in Figure 17, co-culture of NKL cells with anti-GZMB AuNPs and anti-GZMB PLGA/DOX/Ch/ICG NPs does not induce significant changes in IFN-$\gamma$ production while in the case of TNF-a, the anti-GZMB AuNPs induce a significant increase in the production of this cytokine (Kruskal-Wallis test with Dunn's *post-hoc* test; (*) p>0.05 with respect to the IL-15 control group, n=6). Although in the case of IFN-$\gamma$ is observed a slight tendency to increase with the treatment with anti-GZMB AuNPs, it is in the case of TNF-$\alpha$ where a significant increase in the production of the cytokine is observed, however, this tendency to increase with AuNPs can be considered beneficial in a therapeutic scenario, since the production of both cytokines can be associated with the recruitment and activation of other cells of the immune system in the tumor microenvironment. Regarding the results obtained from the production of cytokines in the presence of activating stimuli shown in Figure 18, it is found that, as in Figure 17, the loading of NKL cells with AuNPs induces a slight increase in the production of both cytokines, observing significant differences in the production of IFN-$\gamma$ (Kruskal-Wallis test with Dunn's *post-hoc* test; (*) p>0.05 with respect to the IL-15 control group). Regarding the production of TNF-a, it is found that the loading of PLGA NPs in NKL cells induces a significant decrease in the production of this cytokine (Kruskal-Wallis test with Dunn's *post-hoc* test; (*) p> 0.05 with respect to the IL-15 control group, n=6), however, this decrease in production could be considered negligible because in the control group and the group loaded with AuNPs, the increase is only double with respect to the resting state observed in Figure 17.

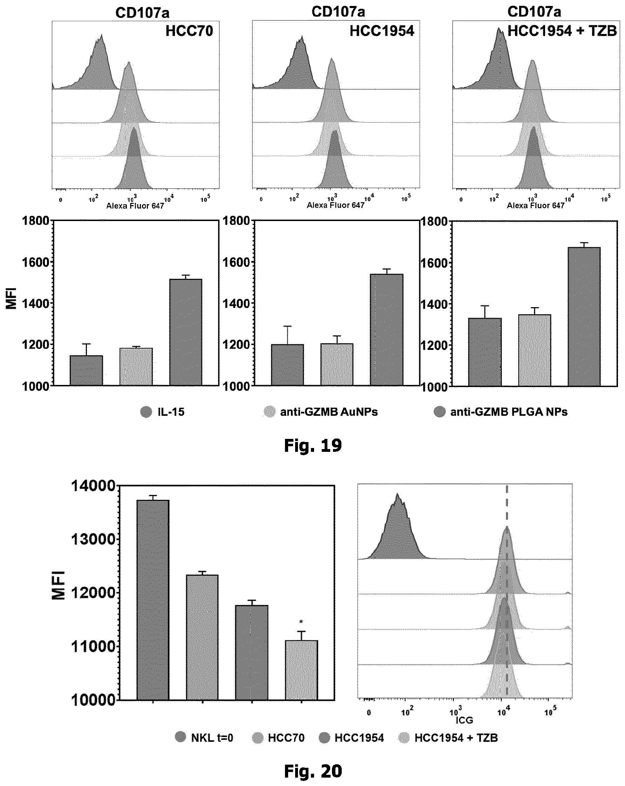

Degranulation assays were performed by culturing 100,000 triple negative (HCC70) or HER2+ (HCC1954) breast cancer cells in 6-well culture plates, under the same culture conditions indicated in Example 8. After 24 hours, the NKL cells were co-cultured with 1,000,000 NKL cells loaded with anti-GZMB AuNPs or anti-GZMB PLGA/DOX/Ch/ICG NPs and after 4 hours of co-culture, was quantified the fluorescence intensity of the degranulation marker in NKL cells, LAMP-1 (CD107a). To assess the influence of CD16 and the induction of dependent cytotoxicity of antibodies in the degranulation event, HER2+ cells were pre-incubated with 30 $\mu$g/mL of Trastuzumab® (TZB) 30 min before cytotoxicity assays. After 4 hours of co-culture, the NKL cells were recovered, washed twice with staining buffer (Biolegend®) and proceeded to stain the membrane for CD107a according to the manufacturer's instructions and proceeded to analyze cells in the flow cytometer. The antibody used for this analysis was Alexa Fluor® 647 anti-human CD107a (LAMP-1) clone H4A3. The results obtained for this test are shown in Figure 19.

As shown in Figure 19, loading of anti-GZMB AuNPs and anti-GZMB PLGA/DOX/Ch/ICG NPs does not induce significant changes in CD107a expression after co-culture with cells HCC70, HCC1954 or HCC1954 + TZB (Kruskal-Wallis test with Dunn's *post-hoc* test; (*) p>0.05 with respect to the IL-15 control group, n=6). In all three cases, it is evident that NKL cells loaded with PLGA NPs exhibit a slightly higher average expression of this exocytosis marker, however, this difference, which is around 400 fluorescence units, can be considered as an advantage in a therapeutic scenario, since an increase in degranulation is related to a higher cytotoxic activity. Regarding the effect of CD16 on degranulation against HCC1954 cells, it is possible to observe that, in all cases, NKL cells increase degranulation against this cell line in the presence of TZB, indicating that the degranulation mechanisms in cells in the absence and presence of CD16 are not negatively altered by the charge of both types of nanoparticles. To corroborate the above, the ICG fluorescence intensity was evaluated in NKL cells loaded with PLGA NPs before (t = 0) and after exposure to tumor cells. As shown in Figure 20, after 4 hours of co-culture with HCC70, HCC1954, and HCC1954+TZB cells, a decrease in fluorescence intensity from ICG is observed, indicating that NKL cells are capable of degranulating NPs after co-culture with triple-negative breast cancer cells and HER2, and it is also shown that the CD16 receptor significantly favors the degranulation events of nanoparticles towards tumor cells in our model (Kruskal-Wallis test with Dunn's *post-hoc* test; (*) p>0.05 with respect to the control group t=0, n=3).

Taken together, the results shown in Figures 16, 17, 18, 19, and 20 indicate that the loading of anti-GZMB AuNPs and anti-GZMB PLGA/DOX/Ch/ICG NPs in NKL cells do not induce alterations in recognition abilities of tumor cells or their effector mechanisms.

**EXAMPLE 13**

In this example, the ideal time for loading anti-GZMB AuNPs and anti-GZMB PLGA/DOX/Ch/ICG NPs obtained in Examples 1 and 4 into NKL cells in accordance with two embodiments of the present invention was evaluated.

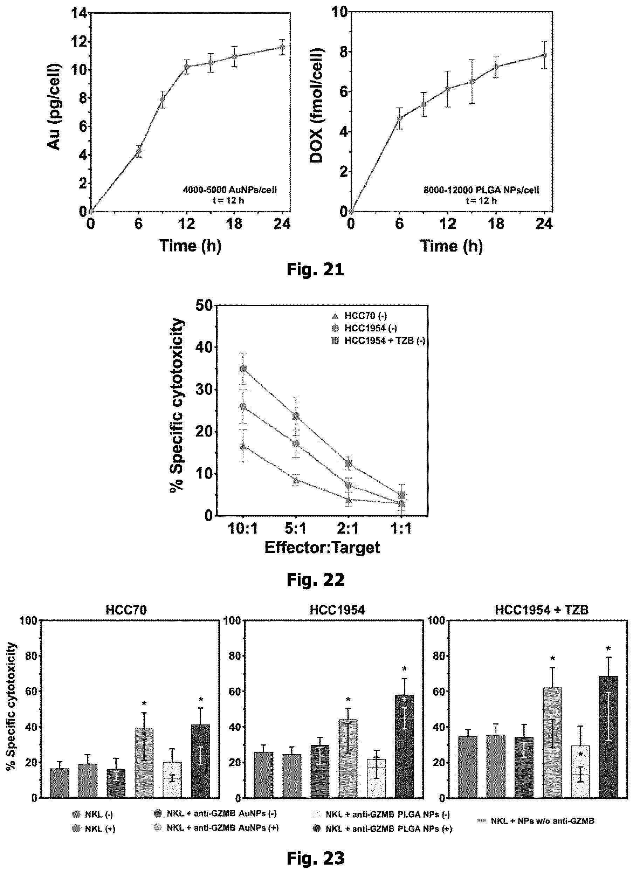

This was done by cultivating $1 \times 10^7$ NKL cells in 75 cm² culture bottles (Nunc®) at a density of 400,000 cells/mL in RPMI-1640 culture medium added with 1% fetal bovine serum (Gibco©), streptomycin a 100 $\mu$g/mL and penicillin at 100 U/mL (Sigma-Aldrich® - P4333) and recombinant human interleukin 15 (IL-15) at 20 ng/mL (Biolegend® - 570304), adding the culture medium with 10.0 $\mu$M of anti-GZMB AuNPs or 50.0 nM of anti-GZMB PLGA NPs and the bottles were incubated for 6, 9, 12, 15, 18, and 24 h. After the incubation time, the NK cells were washed twice with PBS and the pellet was lysed with Triton X-100 in a final volume of 250 $\mu$L. After cell lysate, AuNPs or PLGA NPs quantification was performed according to Example 11. The quantification results were used to estimate the nominal number of nanoparticles present in each cell at each time cut-off and the internalization curves shown in Figure 21 were constructed. The results obtained indicate that the AuNPs have a nominal weight of 0.00228 pg/NP while the PLGA/DOX/Ch/ICG NPs have a nominal weight of 0.000542 pg/NP. The amount of NPs internalized by NKL cells was reported as pg Au/cell for AuNPs and fmol DOX/cell for PLGA NPs.

As can be seen in Figure 21, NKL cells internalize a greater amount of anti-GZMB PLGA/DOX/Ch/ICG NPs compared to anti-GZMB AuNPs, which is consistent with what is observed in Figures 12 and 13. In both cases, it is observed that the internalization reaches a plateau at 15 hours in the AuNPs and at 18 hours in the PLGA NPs, however, for practical purposes and to avoid overloading the NK cells with nanoparticles, the subsequent assay were carried out loading NKL cells with both types of nanoparticles for 12 hours allowing dosing around 4000-5000 AuNPs and 8000-12000 PLGA NPs in each NKL cell.

**EXAMPLE 14**

In this example, was evaluated the basal cytotoxicity profile of NKL cells loaded against triple negative (HCC70) and HER2+ (HCC1954) breast cancer cells at different ratios of effector cells and target cells according to two embodiments of the present invention.

To do the above, 50,000 HCC70 or HCC1954 cells were seeded in 24-well culture plates under the culture conditions indicated in Example 8. After 24 hours, the culture medium was

$$\% \text{ specific cytotoxicity} = \frac{100(\text{Experimental value} - \text{LDH control effector cell} - \text{LDH Control target cell})}{\text{Maximal target cell} - \text{LDH Control target cell}}$$

removed and 50,000, 100,000, 250,000 or 500,000 NKL cells were added in 1 mL of RPMI-1640 culture medium added with 5% fetal bovine serum (Gibco©), 100 $\mu$g/mL treptomycin, and 100 U/mL penicillin (Sigma-Aldrich® - P4333), recombinant human interleukin 2 (IL-2) at 20 ng/mL (Biolegend® - 589104), and recombinant human interleukin 15 (IL-15) at 20 ng/mL (Biolegend® - 570304). To assess the influence of CD16 and the induction of dependent cytotoxicity of antibodies in the cytotoxicity assay, HER2+(HCC1954) cells were pre-incubated with 30 $\mu$g/mL Trastuzumab® (TZB) 30 min before cytotoxicity assays. Following the addition of effector cells to the target cells, the plate was incubated for 4 hours. After 4 h, the plates were centrifuged at 500 $g$, an aliquot of the supernatant was taken and LDH activity was colorimetrically quantified with the Pierce™ LDH cytotoxicity kit (Thermo Scientific) according to the manufacturer's instructions. The specific cytotoxicity in target cells was estimated with the following formula:

LDH activity values of control groups and maximum target cell LDH release were determined from lysis of control wells treated with Triton X-100. The results obtained from the basal cytotoxicity assays are shown in Figure 22. As can be seen, in all cases, the highest cytotoxic activity against HCC70 cells and HCC1954 cells in the absence and presence of TZB corresponds to the effector celltarget cell ratio of 10:1; therefore, the subsequent cytotoxicity assays will be carried out at this proportion.

**EXAMPLE 15**

In this assay, was evaluated the cytotoxic activity of NKL cells loaded with anti-GZMB AuNPs and anti-GZMB PLGA/DOX/Ch/ICG NPs on triple-negative (HCC70) and HER2+ (HCC1954) breast cancer cells in the absence and presence of irradiation with infrared light according to two embodiments of the present invention.

To do the above, 50,000 HCC70 or HCC1954 cells were seeded in 24-well culture plates under the culture conditions indicated in Example 8. After 24 hours, the culture medium was removed and 500,000 NKL cells were added in 1 mL of RPMI-1640 culture medium added with 5% fetal bovine serum (Gibco©), streptomycin at 100 $\mu$g/mL and penicillin at 100 U/mL (Sigma-Aldrich® - P4333), recombinant human interleukin 2 (IL-2) at 20 ng/mL (Biolegend® - 589104) and recombinant human interleukin 15 (IL-15) at 20 ng/mL (Biolegend® -570304). To assess the influence of CD16 and the induction of dependent cytotoxicity of antibodies in the cytotoxicity assay, HER2+(HCC1954) cells were pre-incubated with 30 $\mu$g/mL Trastuzumab® (TZB) 30 min before cytotoxicity assays.

Following the addition of effector cells to the target cells, the plate was incubated for 4 hours. After 4 hours, the wells subjected to irradiation with infrared light were irradiated for 15 min at 1.42 W/cm$^2$ using an optical fiber irradiating only the bottom of the well. After irradiation, an aliquot of the supernatant of the irradiated (+) and non-irradiated (-) groups was taken and the LDH activity was colorimetrically quantified with the Pierce™ LDH cytotoxicity kit (Thermo Scientific) according to the manufacturer's instructions. Specific cytotoxicity on target cells in this cytotoxicity assay was determined using the following formula:

$$\% \text{ specific cytotoxicity} = \frac{100(\text{Experimental value}^* - \text{LDH control effector cell}^* - \text{LDH Control target cell} - \text{Abs NPs})}{\text{Maximal target cell LDH release} - \text{LDH Control target cell}^*}$$

Asterisks in the formula denote the control well being irradiated under the same conditions as the control wells. LDH activity values of control groups and maximal target cell LDH release were determined from lysis of control wells treated with Triton X-100. It is important to note that the effector cells were pre-stimulated with IL-15 for at least 96 hours before performing the cytotoxicity assays, and the loading of nanoparticles with and without anti-GZMB antibodies was performed for 12 hours prior to carry out the cytotoxicity assays. The results obtained from this cytotoxicity assay are shown in Figure 23.

As can be seen, treatments with NKL cells loaded with anti-GZMB AuNPs and anti-GZMB PLGA/DOX/Ch/ICG NPs in combination with irradiation with infrared light (+) significantly increase the cytotoxic activity against tumor cells with respect to the irradiated control group corresponding to NKL cells without NPs in co-culture with tumor cells (one-way ANOVA with Bonferroni *post-hoc* test; (*) p>0.05 with respect to the control group NKL (+)); In particular, the cytotoxicity against HCC70 cells increases by 20% in the treatment with anti-GZMB AuNPs, while the treatment with anti-GZMB PLGA NPs reaches a 25% increase in cytotoxicity. Regarding the HCC1954 cells, the loading with AuNPs increases cytotoxicity by 24% while loading with PLGA NPs by 35%, while in the groups treated with TZB, specific cytotoxicity increases by 15% in both treatments. In the same way, it is possible to observe that treatments with NKL cells loaded with anti-GZMB AuNPs and anti-GZMB PLGA NPs in the absence of irradiation (-) behave practically the same as treatments with NKL cells without nanoparticles, which confirms that the nanoparticle loading does not induce significant effects on the basal cytotoxicity of NKL cells loaded with nanoparticles. Finally, it is possible to appreciate that the treatments with NKL loaded with AuNPs or PLGA NPs without anti-GZMB antibodies are not capable of increasing the specific cytotoxicity against tumor cells (except the groups NKL + anti-GZMB AuNPs (+) vs HCC70 and NKL + anti-GZMB PLGA NPs (+) VS HCC1954), which confirms that functionalization with anti-GZMB monoclonal antibodies allows the localization of AuNPs and PLGA NPs specifically in cytotoxic granules and thus, NKL cells can act as carriers of nanoparticles. Taken together, these results indicate that NKL cells are capable of carrying anti-GZMB AuNPs and anti-GZMB PLGA/DOX/Ch/ICG NPs towards tumor cells and that, in general, this therapeutic strategy allows combining the biological specificity of NKL cells and the therapeutic activity of both types of nanoparticles in a spatio-temporal manner, allowing the basal cytotoxic activity of these cells to be increased by more than 20%.

**EXAMPLE 16**

In this example, is confirmed by flow cytometry the release specificity of anti-GZMB PLGA/DOX/Ch/ICG NPs towards triple negative (HCC70) and HER2+ (HCC1954) breast cancer cells in accordance with one embodiment of the present invention.

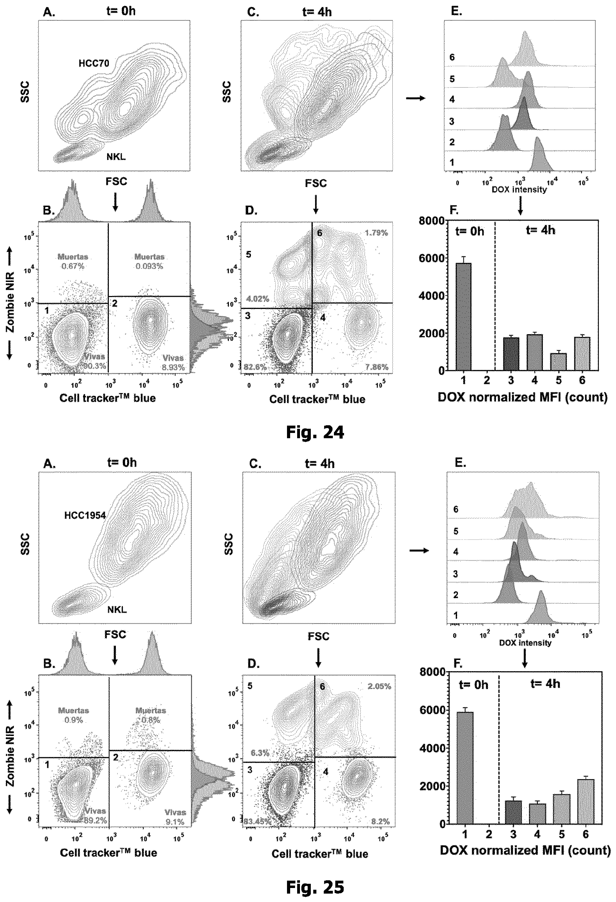

To do the above, 100,000 HCC70 or HCC1954 cells were cultured in 6-well culture plates in RPMI-1640 medium added with 10% fetal bovine serum for 24 hours. After 24 hours, the culture medium was replaced with culture medium without fetal bovine serum added with 0.001 mg/mL of CellTracker™ Blue CMF2HC dye (Invitrogen™) and the cells were incubated for 60 min. After the incubation time, the cells were washed once with PBS, and 1,000,000 NKL cells loaded with anti-GZMB PLGA/DOX/Ch NPs in 3 mL of culture medium were added to the wells under the culture conditions indicated in Example 15. Following the addition of effector cells to the HCC70 and HCC1954 cell wells, the plates were incubated for 4 hours. Because in this assay the intrinsic fluorescence of DOX contained in the NPs is used to verify internalization in target cells, in this assay the cells were not irradiated after the cytotoxicity assay. After 4 hours, the NKL cells from the problem wells were transferred to cytometry tubes, and the remaining tumor cells in the wells were detached with Accutase® (Sigma-Aldrich® - A6964) and were transferred to their respective problem tubes after ensuring the complete detachment. After detachment of the tumor cells, the cells were washed with staining buffer (Biolegend®) and the cells were resuspended in 100 $\mu$L of PBS and 1 $\mu$L of the Zombie NIR™ viability probe (Biolegend®) was added, and the cells were incubated for 30 min in the dark at room temperature. After the incubation time, the cells were fixed with FluoroFix buffer (Biolegend®) for 30 min in the dark at room temperature, and after the incubation time, the cells were washed twice with staining buffer, and the cells were analyzed in the flow cytometer. The results of this assay are shown in Figures 24, 25, 26, and 27.

In this assay it is possible to evaluate the specific cytotoxicity of NKL cells against tumor cells, the degranulation of effector cells, and the internalization of NPs in tumor cells, for which the following elements were identified in the analysis:

- Cell tracker™ blue Cells (-) → NKL Cells
- Cell tracker™ blue Cells (+) → Tumor Cells HCC70 o HCC1954
- Zombie NIR Cells (-) → Living Cells
- Zombie NIR Cells (+) → Dead Cells

From the above, 6 relevant populations can be distinguished for the interpretation of the analysis, in which the intensity of DOX fluorescence was evaluated in order to evaluate the degranulation of effector cells and, internalization in target cells:

    **1.** Living NKL cells (t=0)
    **2.** Living tumor cells (t=0)
    **3.** Living NKL cells (t=4 h)
    **4.** Living tumor cells (t=4h)
    **5.** Dead NKL cells (t=4h)
    **6.** Dead tumor cells (t=4h)

Regarding the cytotoxicity assay in HCC70 cells shown in Figure 24 and what is shown in boxes E and F, it can be concluded that NKL cells are indeed capable of degranulating PLGA NPs against triple-negative breast cancer cells, observing that dead tumor cells have nanoparticles inside. In the same way, living tumor cells exhibit the presence of nanoparticles, suggesting that NKL cells degranulate nonspecifically after their activation.

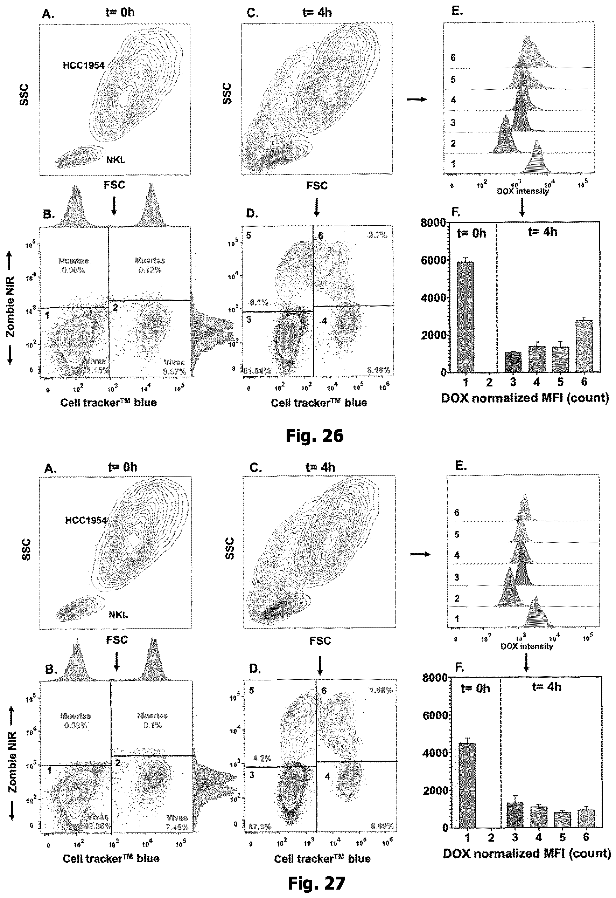

Regarding the assays with HCC1954 and HCC1954+TZB cells shown in Figures 25 and 26, these allow observing a greater internalization of NPs in target cells, which can be explained by the susceptibility to cytotoxic activity by NKL cells in this cell line. In the particular case of Figure 26, it can be seen that the effect of CD16 favors greater internalization of nanoparticles in target cells. Regarding the assay with NKL cells loaded with PLGA NPs without anti-GZMB antibody shown in Figure 27, firstly, it shows a low internalization of PLGA NPs at t=0 and after 4 hours of the cytotoxicity assay, the internalization in target cells is significantly lower than in the previously mentioned groups, which confirms that the functionalization of the nanoparticles with anti-GZMB antibodies increases the accumulation of nanoparticles in NKL cells during the loading process and that it also favors the release of nanoparticles towards tumor cells since it allows that the nanoparticles are mainly located in the cytotoxic granules.

This increase in the cytotoxic activity of NK cells represents an additional biological advantage to the transport of nanoparticles toward tumors, offering great potential for successful application in cancer treatment.

In accordance with the previously described, it will be evident to a person skilled in the art that the preferred embodiment illustrated above is presented for illustrative purposes only, but not limiting the present invention, since a person skilled in the art can make numerous variations of it, as long as they are designed in accordance with the principles of the present invention. As a consequence of the foregoing, the present invention includes all the modalities that a person skilled in the art can propose based on the concepts contained in this description, in accordance with the following claims.

## Claims

1. A stimulated-release photoresponsive nanoparticle, **characterized in that** it comprises a drug encapsulated in a biodegradable synthetic polymer coated with a photosensitizing agent.

2. The stimulated-release photoresponsive nanoparticle according to claim 1, further **characterized in that** it has an average size between 50 nm and 200 nm.

3. The stimulated-release photoresponsive nanoparticle according to claim 1, further **characterized in that** the drug is selected from doxorubicin, cisplatin, carboplatin, or a combination of the above.

4. The stimulated-release photoresponsive nanoparticle according to claim 1, further **characterized in that** the encapsulated drug is between 1% and 25% by weight relative to the weight of the stimulated-release photoresponsive nanoparticle.

5. The stimulated-release photoresponsive nanoparticle according to claim 1, further **characterized in that** the biodegradable synthetic polymer of the stimulated-release photoresponsive nanoparticles is a Poly(lactic-co-glycolic acid) (PLGA) copolymer.

6. The stimulated-release photoresponsive nanoparticle according to claim 1, further **characterized in that** the photosensitizing agent is indocyanine green (ICG).

7. A method for obtaining stimulated-release photoresponsive nanoparticles, **characterized in that** it comprises a stage for forming biodegradable polymeric nanoparticles with encapsulated drugs and a stage for coating said nanoparticles with a photosensitizing agent.

8. The method for obtaining stimulated-release photoresponsive nanoparticles according to claim 7, further **characterized in that** the stage for forming the biodegradable polymeric nanoparticles with encapsulated drugs comprises the following steps: 1) dissolving the biodegradable synthetic polymer with a drug in an organic solvent; 2) adding the organic phase containing the polymer and the drug to an aqueous phase; 3) forming an emulsion; 4) evaporating the solvent; 5) centrifuging the mixture; and 6) washing with deionized water to remove excess of stabilizer and the unencapsulated drug.

9. The method for obtaining stimulated-release photoresponsive nanoparticles according to claim 8, further **characterized in that** the organic solvent is selected from dichloromethane, chloroform, and acetone.

**10.** The method for obtaining stimulated-release photoresponsive nanoparticles according to claim 8, further **characterized in that** the aqueous phase contains a stabilizer.

**11.** The method for obtaining stimulated-release photoresponsive nanoparticles according to claim 10, further **characterized in that** the stabilizer is selected from polyvinyl alcohol or copolymers, or mixtures of copolymers containing polyethylene glycol and polypropylene glycol.

**12.** The method for obtaining stimulated-release photoresponsive nanoparticles according to claim 8, further **characterized in that** the emulsion is formed by sonication.

**13.** The method for obtaining stimulated-release photoresponsive nanoparticles according to claim 8, further **characterized in that** the evaporation of the solvent is carried out by stirring at room temperature.

**14.** The method for obtaining stimulated-release photoresponsive nanoparticles according to claim 7, further **characterized in that** the stage for coating the biodegradable polymeric nanoparticles with encapsulated drugs with a photosensitizing agent comprises the following steps: 1) inverting the surface charge of the nanoparticles to achieve electrostatic physisorption through the formation of a cationic polymer layer leaving -NH$_2$ groups exposed on the surface of the biodegradable polymeric nanoparticles with encapsulated drugs; 2) mixing the biodegradable polymeric nanoparticles with encapsulated drugs with a photosensitizing agent; 3) stimulating the interaction of biodegradable polymeric nanoparticles with encapsulated drugs with the photosensitizing agent by moderate stirring; and 4) removing excess of the photosensitizing agent by dialysis.

**15.** The method for obtaining stimulated-release photoresponsive nanoparticles according to claim 14, further **characterized in that** the biodegradable polymeric nanoparticles with encapsulated drugs and the photosensitizing agent are mixed in a ratio between 0.2% and 5%.

**16.** The method for obtaining stimulated-release photoresponsive nanoparticles according to claim 14, further **characterized in that** the formation of the cationic polymer layer leaving -NH$_2$ groups exposed on the surface of the biodegradable polymeric nanoparticles with encapsulated drugs is carried out by the layer-by-layer method.

**17.** The method for obtaining stimulated-release photoresponsive nanoparticles according to claim 14, further **characterized in that** the cationic polymer is selected from chitosan, cationic cellulose, poly(allylamine) or other cationic polymers, synthetic or natural.

**18.** A biocompatible stimulated-release photoresponsive nanoparticle, **characterized in that** it comprises a stimulated-release photoresponsive nanoparticle coupled to at least one humanized IgG-type monoclonal antibody.

**19.** The biocompatible stimulated-release photoresponsive nanoparticle according to claim 18, further **characterized in that** the humanized IgG-type monoclonal antibody is selected from humanized IgG-type monoclonal antibodies specific for LAMP1 or granzyme B, or a mixture thereof.

**20.** A method for obtaining biocompatible stimulated-release photoresponsive nanoparticles, **characterized in that** it comprises binding humanized IgG-type monoclonal antibodies to stimulated-release photoresponsive nanoparticles by a covalent bond.

**21.** The method for obtaining biocompatible stimulated-release photoresponsive nanoparticles according to claim 20, further **characterized in that** the stimulated-release photoresponsive nanoparticles bind to the humanized IgG-type monoclonal antibodies through the NH$_2$ functional groups.

**22.** The method for obtaining biocompatible stimulated-release photoresponsive nanoparticles according to claim 20, further **characterized in that** the -COOH ends of the humanized IgG-type monoclonal antibodies are preactivated prior to their binding with the biocompatible stimulated-release photoresponsive nanoparticles.

**23.** The method for obtaining biocompatible stimulated-release photoresponsive nanoparticles according to claim 20, further **characterized in that** the preactivation of the -COOH ends of the antibody is carried out by adding 1-ethyl-3-(3-dimethylaminopropyl) carbodiimide (EDC) and sulfo-N-hydroxysuccinimide (sulfo-NHS) to an antibody solution.

**24.** The method for obtaining biocompatible stimulated-release photoresponsive nanoparticles according to claim 20, further **characterized in that** the binding reaction is caused by mixing the stimulated-release photoresponsive nanoparticles with the humanized IgG-type monoclonal antibodies in a ratio of nanoparticle:antibody of 1:1 to 1:20.

25. NK cells, **characterized in that** they comprise cytotoxic granules loaded with biocompatible stimulated-release photoresponsive nanoparticles.

26. The NK cells according to claim 25, further **characterized in that** the NK cells are extracted from the peripheral blood of a patient.

27. A method for introducing biocompatible stimulated-release photoresponsive nanoparticles to the cytotoxic granules of an NK cell, **characterized in that** it comprises incubating NK cells under standard culture conditions in the presence of biocompatible stimulated-release photoresponsive nanoparticles.

28. The method for introducing biocompatible stimulated-release photoresponsive nanoparticles to the cytotoxic granules of an NK cell according to claim 27, further **characterized in that** the standard culture conditions are 37°C, 5% of $CO_2$, in sterile conditions.

29. The method for introducing biocompatible stimulated-release photoresponsive nanoparticles to the cytotoxic granules of an NK cell according to claim 27, further **characterized in that** the incubation of the biocompatible stimulated-release photoresponsive nanoparticles and the NK cells is carried out for 12 hours.

30. An injectable suspension, **characterized in that** it comprises NK cells with cytotoxic granules loaded with biocompatible stimulated-release photoresponsive nanoparticles and a suspension medium.

31. The injectable suspension according to claim 30, further **characterized in that** the suspension medium is an injectable saline solution.

32. A method for obtaining an injectable suspension comprising NK cells with cytotoxic granules loaded with biocompatible stimulated-release photothermal nanoparticles, **characterized in that** it comprises a stage of molecular stimulation of NK cells with cytotoxic granules loaded with biocompatible stimulated-release photoresponsive nanoparticles to obtain activated NK cells; and a stage of mixing the activated NK cells with a suspension medium.

33. The method for obtaining an injectable suspension comprising NK cells with cytotoxic granules loaded with biocompatible stimulated-release photothermal nanoparticles according to claim 32, further **characterized in that** the stage of molecular stimulation of NK cells with cytotoxic granules loaded with biocompatible stimulated-release photothermal nanoparticles comprises adding interleukins to the culture medium.

34. The method for obtaining an injectable suspension comprising NK cells with cytotoxic granules loaded with biocompatible stimulated-release photothermal nanoparticles according to claim 33, further **characterized in that** the interleukins are selected from IL-2, IL-15, IL-18, or mixtures thereof.

35. The method for obtaining an injectable suspension comprising NK cells with cytotoxic granules loaded with biocompatible stimulated-release photothermal nanoparticles according to claim 33, further **characterized in that** the molecular stimulation of NK cells with interleukins is carried out for 72 hours at 37°C and 5% of $CO_2$ under standard cell culture conditions.

36. A method for depositing biocompatible stimulated-release photoresponsive nanoparticles in tumor tissue, **characterized in that** it comprises a stage of obtaining an injectable suspension comprising activated NK cells with cytotoxic granules loaded with biocompatible stimulated-release photothermal nanoparticles; a stage of contacting the activated NK cells with cytotoxic granules loaded with biocompatible stimulated-release photothermal nanoparticles with the tumor tissue, where the activated NK cells with cytotoxic granules loaded with biocompatible stimulated-release photothermal nanoparticles are administered by the injectable suspension; and a stage of releasing the cytotoxic granules loaded with biocompatible stimulated-release photoresponsive nanoparticles contained in the activated NK cells.

37. A method for the release of a drug from a biocompatible stimulated-release photoresponsive nanoparticle, **characterized in that** it comprises exposing the biocompatible stimulated-release photoresponsive nanoparticle to a source of electromagnetic radiation until obtaining an increase in temperature of 23 to 25°C.

**38.** The method for the release of a drug from a biocompatible stimulated-release photoresponsive nanoparticle according to claim 37, further **characterized in that** the source of electromagnetic radiation is a laser.

**39.** The method for the release of a drug from a biocompatible stimulated-release photoresponsive nanoparticle according to claim 38, further **characterized in that** the laser has a wavelength close to 800 nm.

**40.** Use of biocompatible stimulated-release photoresponsive nanoparticles for treating cancerous tumors.

**41.** Use according to claim 40, further **characterized in that** it comprises the deposition of biocompatible stimulated-release photoresponsive nanoparticles in tumor tissue; and the exposure thereof to a source of electromagnetic radiation to induce the release of the drug contained in the nanoparticles into that tumor tissue.

**42.** NK cells, **characterized in that** they comprise cytotoxic granules loaded with biocompatible stimulated-release photoresponsive nanoparticles and biocompatible metallic nanoparticles.

**43.** The NK cells according to claim 42, further **characterized in that** the metal of the biocompatible metallic nanoparticles is gold.

**44.** The NK cells according to claim 42, further **characterized in that** the biocompatible metallic nanoparticles have a spheroid morphology with an average diameter between 50 and 200 nm.

**45.** The NK cells according to claim 42, further **characterized in that** the biocompatible metallic nanoparticles have a hollow interior and the thickness of the metal nanoshell thereof is between 5 and 30 nm.

**46.** The NK cells according to claim 42, further **characterized in that** the biocompatible metallic nanoparticles have a resonance plasmon located at $800 \pm 10$ nm.

EP 4 101 471 A1

Fig. 1

Fig. 2

26

**Fig. 3**

**Fig. 4**

**Fig. 5**

**Fig. 6**

**Fig. 7**

Fig. 8

Fig. 9

Fig. 10

Fig. 11

**Fig. 12**

**Fig. 13**

**Fig. 14**

Fig. 15

Fig. 16

Fig. 17

Fig. 18

**Fig. 19**

**Fig. 20**

**Fig. 21**

**Fig. 22**

**Fig. 23**

Fig. 24

Fig. 25

Fig. 26

Fig. 27

# EP 4 101 471 A1

**INTERNATIONAL SEARCH REPORT**

| | |
|---|---|
| International application No. | |
| | PCT/IB2020/061716 |

**A. CLASSIFICATION OF SUBJECT MATTER**

CIP: A61K47/59, A61K31/704, A61K31/282, A61K45/08, A61K41/00, A61K9/51, A61P35/00, B82Y5/00 (2021.01)

According to International Patent Classification (IPC) or to both national classification and IPC

**B. FIELDS SEARCHED**

Minimum documentation searched (classification system followed by classification symbols)

Documentation searched other than minimum documentation to the extent that such documents are included in the fields searched

Electronic data base consulted during the international search (name of data base and, where practicable, search terms used)

STN (USPATFULL, BIOTECHNO, BIOSIS, EMBASE), PATENTSCOPE, ESPACENET, GOOGLE SCHOLAR, INAPI

**C. DOCUMENTS CONSIDERED TO BE RELEVANT**

| Category* | Citation of document, with indication, where appropriate, of the relevant passages | Relevant to claim No. |
|---|---|---|
| X<br>---------<br>Y | US 2017/095558 A1 (UNIVERSITY OF SOUTH CAROLINA) 06 April 2017<br>The whole document | 1-5, 7-13, 18, 20, 37-41<br>----------------------<br>6, 14-17, 21-35, 42-46 |
| X | PENG, Y. et al. A multifunctional nanoplatform for cancer chemo-photothermal synergistic therapy and overcoming multidrug resistance. Biomaterials science, 2018; 6(5): 1084-1098. DOI: 10.1039/c7bm01206c<br>Abstract, more information | 1, 2, 5, 7, 37-41 |
| Y | LEE, Y-H & LAI, Y-H. Synthesis, Characterization, and Biological Evaluation of Anti-HER2 Indocyanine Green-Encapsulated PEG-Coated PLGA Nanoparticles for Targeted Phototherapy of Breast Cancer Cells. PLoS ONE, 2016; 11(12): e0168192. 17 pp. DOI: 10.1371/journal.pone.0168192<br>The whole document | 6 |

| ☐ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15/03/2021 | 26/03/2021 |

| Name and mailing address of the ISA/ | Authorized officer |
|---|---|
| INAPI, Av. Libertador Bernardo O'Higgins 194, Piso 17, Santiago, Chile | GARRIDO, Carolina |
| Facsimile No. | Telephone No. |

Form PCT/ISA/210 (second sheet) (January 2015)

**INTERNATIONAL SEARCH REPORT**

| International application No. |
| --- |
| PCT/IB2020/061716 |

**Box No. II     Observations where certain claims were found unsearchable (Continuation of item 2 of first sheet)**

This international search report has not been established in respect of certain claims under Article 17(2)(a) for the following reasons:

1. ☒ Claims Nos.: 36
   because they relate to subject matter not required to be searched by this Authority, namely:

   Claim 36 relates to a subject matter that is considered by this International Searching Authority to be affected by PCT Rule 39.1(iv), concerning methods for treatment of the human or animal body by surgery or therapy, as well as diagnostic methods. In conclusion, no expert opinion will be established in respect of novelty, inventive step and industrial applicability for this claim (PCT Article 17(2)(a)(b)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (January 2015)

**INTERNATIONAL SEARCH REPORT**

| International application No. |
| --- |
| PCT/IB2020/061716 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | DOMÍNGUEZ-RÍOS, R. et al. Cisplatin-loaded PLGA nanoparticles for HER2 targeted ovarian cancer therapy. Colloids and Surfaces B: Biointerfaces, 2019;.178: 199-207. DOI: 10.1016/j.colsurfb.2019.03.011<br>The whole document | 14-17, 21-24 |
| Y | HUANG, S. et al. Nano-loaded natural killer cells as carriers of indocyanine green for synergetic cancer immunotherapy and phototherapy. Journal of Innovative Optical Health Sciences, 2019; 12 (3): 1941002. 8 pp. DOI: 10.1142/S1793545819410025<br>The whole document | 25-35, 42-46 |
| P, X | LIANG, J. et al. Doxorubicin-loaded pH-responsive nanoparticles coated with chlorin e6 for drug delivery and synergetic chemo-photodynamic therapy. Nanotechnology, 21 Feb 2020; 31 (19): 195103. DOI: 10.1088/1361-6528/ab6fd5<br>The whole document | 1-5, 7 |
| A | ANSELMO, A. C. &, MITRAGOTRI S. Cell-mediated delivery of nanoparticles: taking advantage of circulatory cells to target nanoparticles. J Control Release. 2014 Sep 28;190:531-41. DOI: 10.1016/j.jconrel.2014.03.050.<br>The whole document | |
| A | CHAI, F. et al. Doxorubicin-loaded poly (lactic-co-glycolic acid) nanoparticles coated with chitosan/alginate by layer by layer technology for antitumor applications. International Journal of Nanomedicine, 2017; 12: 1791-1802. DOI:10.2147/IJN.S130404<br>The whole document | |
| A | LU, B. et al. Chitosan-Modified PLGA Nanoparticles for Control-Released Drug Delivery. Polymers, 12 Feb. 2019; 11:304. 14 pp. DOI:10.3390/polym11020304<br>The whole document | |
| A | MAKSIMENKO, O. et al. Doxorubicin-loaded PLGA nanoparticles for the chemotherapy of glioblastoma: Towards the pharmaceutical development. Int J Pharm. 2019 Dec 15;572:118733. DOI: 10.1016/j.ijpharm.2019.118733. Epub 2019 Nov 2.<br>The whole document | |
| A | SIDDHARTH, S. et al. Chitosan-Dextran sulfate coated doxorubicin loaded PLGA-PVA-nanoparticles caused apoptosis in doxorubicin resistance breast cancer cells through induction of DNA damage. Sci Rep, 2017; 7:2143. 10 pp. DOI: 10.1038/s41598-017-02134-z<br>The whole document | |

Form PCT/ISA/210 (continuation of second sheet) (January 2015)

**INTERNATIONAL SEARCH REPORT**

International application No.

PCT/IB2020/061716

C (Continuation). DOCUMENTS CONSIDERED TO BE RELEVANT

| Category* | Citation of document, with indication, where appropriate, of the relevant passages | Relevant to claim No. |
|---|---|---|
| A | SUN, H. et al. Silibinin and indocyanine green-loaded nanoparticles inhibit the growth and metastasis of mammalian breast cancer cells in vitro. Acta Pharmacol Sin, 2016; 37: 941-949. DOI: 10.1038/aps.2016.20<br>The whole document | |
| A | ZHENG, M. et al. Single-Step Assembly of DOX/ICG Loaded Lipid-Polymer Nanoparticles for Highly Effective Chemo-photothermal Combination Therapy. ACS Nano, 2013; 7(3): 2056-2067. DOI:10.1021/nn400334y<br>The whole document | |
| A | REZVANTALAB, S. et al. PLGA-based nanoparticles in cancer treatment. Frontiers in pharmacology, 2018; 9:1260. 19 pp. DOI: 10.3389/fphar.2018.01260<br>The whole document | |

Form PCT/ISA/210 (continuation of second sheet) (January 2015)

INTERNATIONAL SEARCH REPORT
Information on patent family members

International application No.

PCT/IB2020/061716

| Patent document cited in the search report | Publication date | Patent family member(s) | Publication date |
|---|---|---|---|
| US2017095558 (A1) | 06-04-2017 | US10835605 (B2) | 17-11-2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016145578 A
- EP 2398466 A
- EP 1917004 A
- EP 2736537 A
- EP 2637700 A
- EP 2970907 A
- EP 2508207 A
- WO 2012142669 A
- EP 2680919 A
- EP 1834646 A
- EP 1841468 A